(19) Europäisches Patentamt
European Patent Office
Office européen des brevets

(11) **EP 4 806 985 A1**

(12) **EUROPEAN PATENT APPLICATION**
published in accordance with Art. 153(4) EPC

(43) Date of publication:
**16.09.2026 Bulletin 2026/38**

(21) Application number: **24889056.8**

(22) Date of filing: **04.11.2024**

(51) International Patent Classification (IPC):
***C12N 1/20*** *(2026.01)* ***C12N 1/38*** *(2006.01)*
***C12N 9/52*** *(2006.01)*

(52) Cooperative Patent Classification (CPC):
**C12N 1/20; C12N 1/38; C12N 9/52**

(86) International application number:
**PCT/KR2024/017155**

(87) International publication number:
**WO 2025/100868 (15.05.2025 Gazette 2025/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.11.2023 KR 20230151397**

(71) Applicant: **Daewoong Co., Ltd.**
**Seongnam-si, Gyeonggi-do 13211 (KR)**

(72) Inventors:
- **KIM, Kyoung-Yun**
  **Suwon-si Gyeonggi-do 16695 (KR)**
- **PARK, Jiseong**
  **Hwaseong-si Gyeonggi-do 18623 (KR)**
- **CHOO, Seungjung**
  **Hwaseong-si Gyeonggi-do 18623 (KR)**
- **KIM, Semi**
  **Hwaseong-si Gyeonggi-do 18623 (KR)**

(74) Representative: **Mewburn Ellis LLP**
**Aurora Building**
**Counterslip**
**Bristol BS1 6BX (GB)**

(54) **MEDIUM COMPOSITION FOR PREPARING BOTULINUM TOXIN**

(57) The present invention relates to a medium composition for producing botulinum toxin and, more specifically, to a medium composition for culturing *Clostridium* sp. strains capable of producing botulinum toxin. When a medium containing vegetable peptone, glucose, and yeast extracts and not containing animal-derived components according to the present invention is used for culturing *Clostridium* botulinum strains, the strain growth rate is increased compared to the existing mediums, and the strains can be safely cultured due to not containing animal-derived components, thereby increasing the productivity of botulinum toxin.

Fig.5

OD 540nm
Time (hr)
APF DoE
Media 1
Media 2
Media 3
Media 4
Media 5
Media 6
Media 7
Media 8
Media 9
Media 10
Media 11
Media 12
Media 13
Media 14
Media 15
Media 16

EP 4 806 985 A1

Processed by Luminess, 75001 PARIS (FR)

## Description

[Technical Field]

The present invention relates to a medium composition for production of botulinum toxin, and more specifically, to a medium composition for culturing *Clostridium* sp. strains capable of producing botulinum toxin. The medium composition of the present invention is characterized by containing plant peptones, including wheat peptone and peptone from pea, D-(+)-glucose, and yeast extract.

[Background Art]

A variety of *Clostridium* sp. strains that secrete neurotoxins have been discovered since the 1890s, and characterization of toxins secreted from these strains has been conducted for the past 70 years (Schant, E. J. et al., Microbiol. Rev., 56:80, 1992).

Neurotoxins derived from the *Clostridium* sp. strains, that is, botulinum toxins, are classified into seven types (types A to G) depending on their serological properties. Each of the toxins has a toxin protein having a size of about 150 kDa and naturally contains a complex of several non-toxic proteins. A medium complex (300 kDa) is composed of a toxin protein and a non-toxic non-hemagglutinin protein, and a large complex (450 kDa) and a large-large complex (900 kDa) are composed of the medium complex bound to hemagglutinin (Sugiyama, H., Microbiol. Rev., 44:419, 1980). Such non-toxic hemagglutinin proteins are known to function to protect the toxin from low pH and various proteases in the intestines.

The toxin is synthesized as a single polypeptide having a molecular weight of about 150 kDa in cells, and then is cleaved, at a position 1/3 from the N-terminal end, by the action of intracellular protease or treatment with an artificial enzyme such as trypsin into two units: a light chain (L; molecular weight: 50 kDa) and a heavy chain (H; molecular weight: 100 kDa). The cleaved toxin has greatly increased toxicity compared to the single polypeptide. The two units are linked to each other by a disulfide bond and have different functions. The heavy chain binds to a receptor of a target cell (Park. M. K. et al., FEMS Microbiol. Lett., 72:243, 1990) and functions to interact with a biomembrane at low pH (pH 4) to form a channel (Mantecucco, C. et al., TIBS., 18:324, 1993), and the light chain has pharmacological activity, and interferes with the secretion of a neurotransmitter when cells have been permeabilized using a detergent or the light chain has been introduced into cells by, for example, electroporation (Poulain, B. et al., Proc. Natl. Acad. Sci. USA., 85:4090, 1988).

The toxin inhibits the exocytosis of acetylcholine at the cholinergic presynapse of a neuromuscular junction, causing asthenia. It has been believed that treatment with a very small amount of the toxin exhibits toxicity, suggesting that the toxin has any enzymatic activity (Simpson, L. L. et al., Ann. Rev. Pharmaeol. Toxicol., 26:427, 1986).

According to a recent report, the toxin has metallopeptidase activity, and its substrate is composed of synaptobrevin, syntaxin, a synaptosomal associated protein of 25 kDa (SNAP25) and the like, which are the unit proteins of an exocytosis machinery complex. Each type of toxin uses one of the above-described three proteins as its substrate, and it is known that type B, D, F and G toxins cleave synaptobrevin at a specific site, type A and E toxins cleave SNAP25 at a specific site, and type C toxin cleaves syntaxin at a specific site (Binz, T. et al., J. Biol. Chem., 265:9153, 1994).

Particularly, type A botulinum toxin is known to be soluble in a dilute aqueous solution at a pH of 4.0 to 6.8. It is known that a stable non-toxic protein is separated from neurotoxin at a pH of about 7 or higher, and as a result, the toxicity is gradually lost. Particularly, it is known that the toxicity decreases as pH and temperature increase.

Botulinum toxin is fatal to humans even in small amounts and is easy to produce in large amounts. Thus, it constitutes four major bio-terror weapons together with *Bacillus anthracis, Yersinia pestis,* and smallpox virus. However, it has been found that, when type A botulinum toxin is injected at a dose that does not systematically affect the human body, it can paralyze local muscle around the injection site. Based on this characteristic, type A botulinum toxin can be used in a wide range of applications, including wrinkle removing agents, agents for treating spastic hemiplegia and cerebral palsy, etc. Thus, the demand for type A botulinum toxin has increased, and studies on methods of producing botulinum toxin so as to satisfy the demand have been actively conducted.

A current typical commercial product is BOTOX® (a purified neurotoxin complex of type A botulinum toxin) that is commercially available from Allergan, Inc., USA. A 100-unit vial of BOTOX® is composed of about 5 ng of a purified type A botulinum toxin complex, 0.5 mg of human serum albumin, and 0.9 mg of sodium chloride, is provided in a vacuum-dried form, and is reconstituted using sterile saline without a preservative (injection of 0.9% sodium chloride). Other commercial products include Dysport® (a *Clostridium botulinum* type A toxin-hemagglutinin complex which has lactose and human serum albumin in a pharmaceutical composition containing botulinum toxin and is reconstituted using 0.9% sodium chloride before use) that is commercially available from Ipsen Ltd., UK, MyoBloc® (an injectable solution with a pH of about 5.6 comprising botulinum type B toxin, human serum albumin, sodium succinate, and sodium chloride) that is commercially available from Solstice Neurosciences, Inc.

A medium for culturing a *Clostridium botulinum s*train, which is generally used in a method for production of botulinum toxin as disclosed in Korean Patent No. 10-1339349, contains animal-derived components. Thus, if an animal-

derived abnormal prion known as an agent that causes transmissible spongiform encephalopathy is contained in the animal components due to contamination, it poses problems in a process for producing botulinum toxin.

Transmissible spongiform encephalopathy (TSE) is a type of a neurodegenerative disorder causing serious degeneration of neurons, and examples thereof includes bovine spongiform encephalopathy (BSE), Scrapie, Creutzfeldt-Jakob disease (CJD), Gerstmann-Straussler-Scheinker syndrome, Kuru, transmissible mink encephalopathy, chronic wasting disease, feline spongiform encephalopathy, etc., which affect humans and animals. It was reported that BSE crosses the species barrier and affects even humans.

The agent that causes transmissible spongiform encephalopathy is characterized in that it is non-immunogenic and has a long incubation period. From post-mortem analysis of the brains of cattle infected with bovine spongiform encephalopathy, it can be seen that a unique pattern of spongiform vacuoles was formed in the brain due to the destruction of neurons and the deposition of abnormal protein fibers.

The pathogen believed to be the cause of transmissible spongiform encephalopathy is an infectious protein called an abnormal prion. Unlike regular viruses that require nucleic acids, the abnormal prion is an infectious particle composed only of protein and containing no nucleic acid. Regarding TSE, it is known that, when an abnormal prion (PrPsc) that is an infectious particle binds to a normal prion (PrPc), it is converted to a pathogenic prion which then accumulates in the brain (Prusiner SB, Alzheimer Dis Assoc Disord., 3:52-78, 1989).

Creutzfeldt-Jakob disease is a rare neurodegenerative disorder of human transmissible spongiform encephalopathy (TSE) where the transmissible agent is apparently an abnormal isoform of a prion protein. An individual with Creutzfeldt-Jacob disease can deteriorate from apparent perfect health to akinetic mutism within six months. Thus, when a pharmaceutical composition containing a biologic, such as botulinum toxin, obtained using animal-derived products, there is a risk of developing a prion-mediated disease such as Creutzfeldt-Jakob disease. Thus, if a pharmaceutical composition is prepared using stock produced using animal-derived components, there is a risk that the patient will receive various pathogens or infectious agents.

Accordingly, it has been reported that adding a casein hydrolysate (e.g., TSE-certificated casein hydrolysate) to an animal protein-free (APF) medium to prevent TSE infection improves the growth rate of strains and the production of botulinum toxin (Korean Patent No. 17-1729251, etc.). However, since the APF medium to which the casein hydrolysate has been added still contains casein, which is an animal-derived component, research is needed to achieve high growth rates and high toxin production of *Clostridium botulinum* strains in APF medium that does not contain animal-derived components.

Accordingly, the present inventors have made extensive efforts to develop a medium that contains plant peptones causing no risk of TSE infection when culturing *Clostridium botulinum* strains and does not contain animal-derived components in order to prevent the risk of prion-mediated disease as described above, and as a result, have found that the medium can improve the growth rate of *Clostridium botulinum* strains and the toxin production of the strains compared to conventional media, thereby completing the present invention.

[Disclosure]

[Technical Problem]

An object of the present invention is to provide a medium composition for culturing *Clostridium botulinum* strains that does not contain animal-derived components.

Another object of the present invention is to provide a method of improving the production of botulinum toxin by culturing *Clostridium botulinum* in the above-described medium composition.

[Technical Solution]

To achieve the above objects, the present invention provides a medium composition for culturing *Clostridium botulinum* strains, which contains plant peptones, including wheat peptone and peptone from pea, D-(+)-glucose, and yeast extract, and does not contain animal-derived components.

The present invention also provides a method for producing botulinum toxin, comprising steps of: (a) producing botulinum toxin by culturing *Clostridium botulinum* using the medium composition; and (b) recovering the produced botulinum toxin.

[Advantageous effects]

When the medium according to the present invention, which contains plant peptones, glucose, and yeast extract and does not contain animal-derived components, is used to culture *Clostridium botulinum* strains, the medium may increase the growth rate of the strains compared to conventional media, and increase the production of botulinum toxin by

culturing the strain in a safe manner because it does not contain animal-derived components.

[Description of Drawings]

FIG. 1 shows a JMP custom design to determine the appropriate proportion of plant peptones using three plant peptones (peptone from pea, wheat peptone, and peptone from potatoes No. 2) as factors and an $OD_{540nm}$ value of 3 or higher as a response.
FIG. 2 shows the results of design evaluation by power analysis of the custom design and the fraction of design space plot.

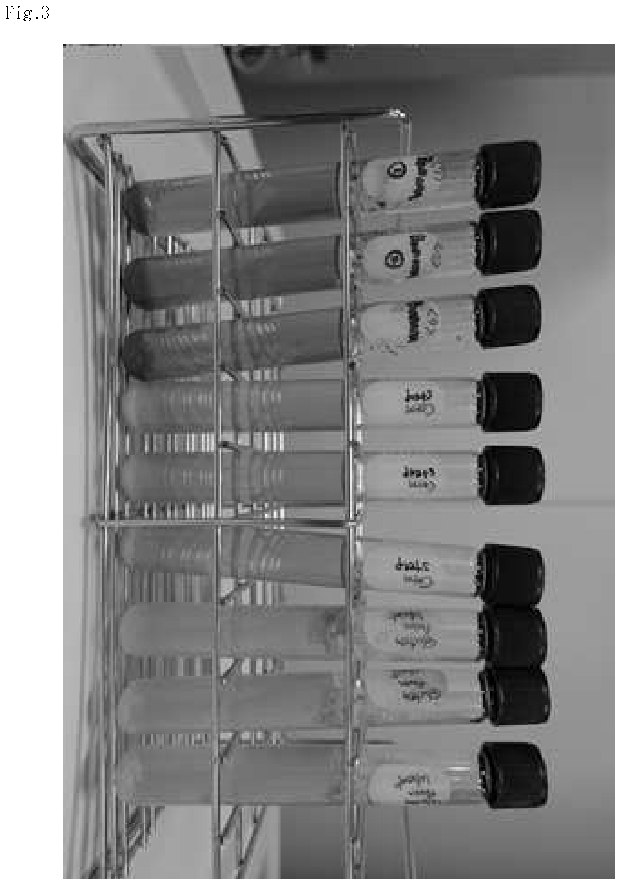

FIG. 3 shows media containing APF medium components.
FIG. 4a is a graph showing the results of measuring the growth rate of a *Clostridium botulinum* strain over time in APF media containing dextrin as a carbon source, and FIG. 4b is a graph showing the results of measuring the growth rate of a *Clostridium botulinum* strain over time in APF media containing glucose as a carbon source.
FIG. 5 is a graph showing the results of measuring the growth rate of a *Clostridium botulinum* strain over time in 16 different types of APF media.
FIG. 6 shows distribution analysis of D-optimal design experiments, and it can be seen that the experimental results are generally evenly distributed.
FIG. 7 shows scatterplot matrix analysis of D-optimal design results, and it can be seen that wheat peptone and peptone from pea have a weak positive correlation with $OD_{540nm}$ and toxin concentration.
FIG. 8 shows the results of outlier analysis, and it can be seen that there are no outliers in the experimental results.
FIG. 9 shows the process for establishing a model of $OD540_{nm}$, and it can be seen that the VIF value does not exceed 10 and is at level 1, suggesting that there is no problem with the model, and that all studentized residuals results are included in the 95% confidence interval.
FIG. 10 shows the prediction formula for $OD_{540nm}$.
FIG. 11 shows the interaction profiles of $OD_{540nm}$.
FIG. 12 shows the process for establishing a model of $OD540_{nm}$, and it can be seen that the VIF value does not exceed 10 and is at level 1, suggesting that there is no problem with the model, and that all studentized residuals results are included in the 95% confidence interval.
FIG. 13 shows the prediction formula for toxin concentration.
FIG. 14 shows interaction profiles.
FIG. 15 shows the process of finding medium conditions for $OD_{540nm}$ and toxin concentration using a Prediction Profiler based on the prediction formula for each response value.
FIGS. 16a and 16b show the distribution of Y in Spec (FIG. 16a) and edge of failure analysis (FIG. 16b) after performing 5,000 simulations as a function of the random variation in the factors and model noise using the simulator function.
FIG. 17 shows the analysis of pea capability with the range corresponding to $3\sigma$ set as NORs and the range corresponding to $4.5\sigma$ set as PARs.
FIG. 18 shows the analysis of wheat capability with the range corresponding to $3\sigma$ set as NORs and the range corresponding to $4.5\sigma$ set as PARs.
FIG. 19 shows the contour profiler results of the APF medium, and it can be seen that, when NORs ($3\sigma$, red line) and PARs ($4.5\sigma$, blue line) were plotted on a contour plot, they did not exceed the white range, which is the range satisfying the model.
FIG. 20 shows the growth curve of a botulinum strain in each APF medium.
FIG. 21 shows the level of toxin expression for each APF medium.

[Best Mode]

Although an animal protein-free (APF) medium composition exhibited an improved growth rate of *Clostridium botulinum* strains compared to conventional media, there is a need to add medium components that further improve the growth rate of the strains without the risk of infection such as TSE infection. Recently, a casein hydrolysate (e.g., TSE-certificated casein hydrolysate) that has not been reported to cause TSE infection has been added to APF medium to grow strains and produce toxins.

However, casein hydrolysate is a component extracted from milk, and casein accounts for about 80% of the total protein contained in milk. Although APF medium containing casein hydrolysate does not contain animal-derived components such as fetal bovine serum (FBS), casein hydrolysate itself is an animal-derived component, and thus there is a need for a highly safe medium for culturing *Clostridium botulinum* strains, which contains plant peptones without containing animal-derived components such as casein hydrolysate.

Accordingly, in one aspect, the present invention relates to a medium composition for culturing *Clostridium botulinum* strains, which contains plant peptones, including wheat peptone and peptone from pea, D-(+)-glucose, and yeast extract, and does not contain animal-derived components. Here, "plant peptone" means peptone extracted from wheat or pea, and preferably may be commercially available Millipore™ 96174, or Millipore™ 93492-500G-F, without being limited thereto.

In the present invention, the term "conventional medium" refers to a medium containing casein hydrolysate, yeast extract, and thioglycolate medium, which are animal-derived components. Additionally, the term "animal protein-free (APF) medium" refers to a medium not containing animal-derived proteins.

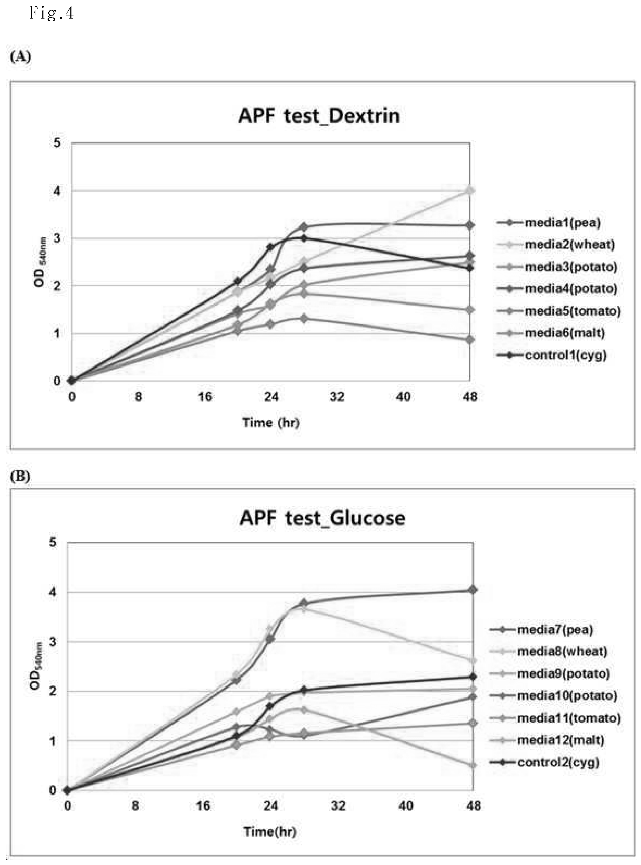

In one example of the present invention, APF media were prepared containing various plant-based medium ingredients, including peptone from pea, wheat peptone, peptone from potatoes No. 2, corn steep solid, gluten from wheat, potato peptone E210, banana powder, tomato juice, and malt extract, without animal-derived components, under transmissible spongiform encephalopathy (TSE)-free conditions, and the growth rates and toxin expression levels of the strain in the media were compared. As a result, it could be confirmed that the plant-derived medium components for efficiently culturing the *Clostridium botulinum* strain included peptone from pea, wheat peptone, and peptone from potatoes No. 2, and in this case, it was confirmed that the growth and toxin expression of the strain were excellent. Finally, as shown in Tables 4 and 5 below and FIG. 4, it was determined that the plant peptones contained in the final selected medium composition for culturing the *Clostridium botulinum* strain were peptone from pea, wheat peptone, and potato peptone.

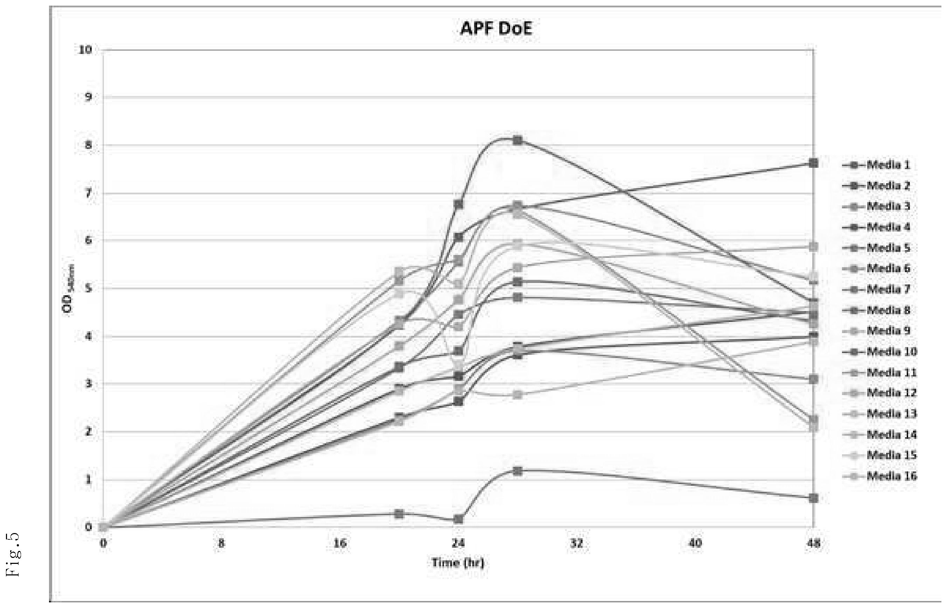

In another example of the present invention, APF media were prepared containing peptone from pea, wheat peptone, and potato peptone at various compositional ratios without animal-derived components under transmissible spongiform encephalopathy (TSE)-free conditions, and the growth rates and toxin expression levels of the strain in the media were compared. As a result, it could be confirmed that the medium composition for efficiently culturing the *Clostridium botulinum* strain contained at least one plant peptone selected from the group consisting of wheat peptone and peptone from pea, in addition to a carbon source (e.g., glucose) and a nitrogen source (e.g., yeast extract), and in this case, the growth and toxin expression of the strain were excellent. Finally, as shown in Tables 8 to 12 below and FIGS. 5 to 19, the contents of plant peptones in the final selected medium composition for culturing the *Clostridium botulinum* strain were 18 g/L of peptone from pea and 7 g/L of wheat peptone.

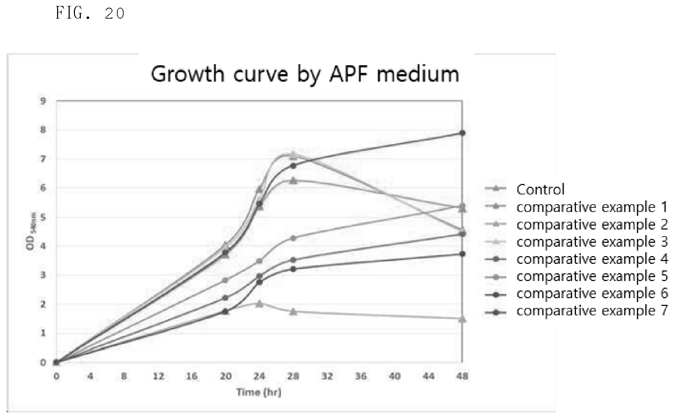

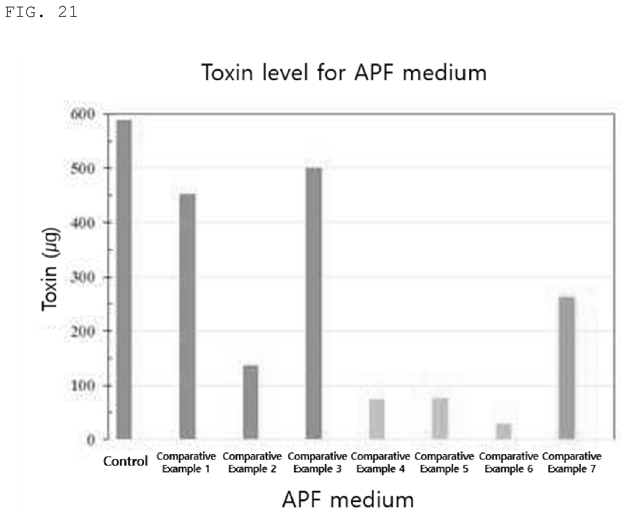

In another example of the present invention, an experiment was conducted to compare the growth pattern and toxin concentration of the *Clostridium botulinum* strain between when grown in an APF medium containing the final selected plant peptone of the present invention and when grown in a medium (Comparative Example 7) described in Korean Patent Application Publication 10-2022-0140696, which was published prior to the present invention. As a result, as shown in Tables 13 to 15 below and FIGS. 20 and 21, in the case of the medium of the present invention, the $OD_{540nm}$ value was 7.1021 after 28 hours of culture of the *Clostridium botulinum* strain, and then the OD value gradually decreased, the $OD_{540nm}$ value was 5.5636 after 48 hours, and the toxin concentration in the supernatant of *Clostridium botulinum* toxin was 588.8 μg (10 mL of culture medium) after 48 hours of culture. Meanwhile, as shown in Tables 13 to 15 below and FIGS. 20 and 21, in the case of the medium of Comparative Example 7, the $OD_{540nm}$ value was 6.7739 after 28 hours of culture of the *Clostridium botulinum* strain, and the toxin concentration in the supernatant of *Clostridium botulinum* toxin was 263.7 μg (10 mL of culture medium) after 48 hours of culture, suggesting the toxin expression level decreased by 55.2% compared to that in the case of the medium of the present invention.

In another embodiment of the present invention, an experiment was conducted to compare the growth pattern and toxin concentration of the *Clostridium botulinum* strain between the medium of the present invention and media (Comparative Examples 1 to 3) further containing L-cysteine hydrochloride monohydrate and/or medical antifoam C emulsion (Dow Corning®), which are/is the compositional component(s) of the medium described in Korean Patent Application Publication No. 10-2022-0140696, in addition to the medium of the present invention. As a result, as shown in Tables 13 to 15 below and FIGS. 20 and 21, it can be confirmed that, in the case of Comparative Examples 1 to 3, the $OD_{540nm}$ value was lower than that in the medium of the present invention, and the toxin expression level after 48 hours of culture decreased by 14.9 to 76.6% compared to that in the medium of the present invention.

In another example of the present invention, an experiment was conducted to compare the growth pattern and toxin concentration of the *Clostridium botulinum* strain between the medium of the present invention and media (Comparative Examples 4 to 6) containing soy peptone in place of peptone from pea and/or wheat peptone in the medium of the present invention and further containing L-cysteine hydrochloride monohydrate and medical antifoam C emulsion (Dow Corning®). As a result, as shown in Tables 13 to 15 below and FIGS. 20 and 21, it can be confirmed that, in the case of Comparative Examples 4 to 6, the $OD_{540nm}$ value was significantly lower than that in the medium of the present invention, and the toxin expression level after 48 hours of culture decreased by 87.0 to 94.9% compared to that in the medium of the present invention.

In the present invention, the medium composition for culturing *Clostridium botulinum* strains may contain at least one plant-derived component selected from the group consisting of peptone from pea, wheat peptone, peptone from potatoes No. 2, corn steep solid, gluten from wheat, potato peptone E210, banana powder, tomato juice, and malt extract,

preferably may contain peptone from pea, wheat peptone, and peptone from potatoes No. 2, and more preferably may contain peptone from pea and wheat peptone, without being limited thereto.

As used herein, the term "plant peptone" or "plant hydrolysate" refer to the digestion products of proteins obtained from plants. For example, wheat peptone (wheat hydrolysate) refers to the product obtained by digesting the total protein obtained from wheat. Also, "casein hydrolysate" refers to the digestion product of casein protein.

The digestion of the plant protein or casein protein is preferably carried out by partial digestion. The digestion of the protein is preferably carried out by acid treatment, base treatment, enzyme treatment, high-pressure treatment, heat treatment, or physical treatment, and more preferably, the plant peptone or casein hydrolysate may be enzyme-treated. The physical treatment is, for example, grinding.

The plant peptone or casein hydrolysate in the present invention is a partial degradation product of protein, and is in the form of a mixture containing not only amino acids as single molecules but also peptides composed of several to tens of amino acids and whole protein molecules.

In the present invention, the content of the plant peptones may be 0.1 to 10 w/v% (1 to 100 g/L), preferably 0.2 to 5 w/v% (2 to 50 g/L), more preferably 0.5 to 2 w/v% (5 to 20 g/L).

In the present invention, the content of the wheat peptone may be 0.1 to 4.0 w/v% (1 to 40 g/L), preferably 0.45 to 0.95 w/v% (4.5 to 9.5 g/L), preferably 0.48 to 0.92 w/v% (4.8 to 9.2 g/L), preferably 0.55 to 0.85 w/v% (5.5 to 8.5 g/L), more preferably 0.7 w/v% (7 g/L).

In the present invention, the content of the pea peptone may be 0.1 to 3.5 w/v% (1 to 35 g/L), preferably 1.55 to 2.05 w/v% (15.5 to 20.5 g/L), preferably 1.58 to 2.03 w/v% (15.8 to 20.3 g/L), preferably 1.65 to 1.95 w/v% (16.5 to 19.5 g/L), more preferably 1.8 w/v% (18 g/L).

In the present invention, the medium composition for culturing *Clostridium botulinum* strains may not contain L-cysteine hydrochloride monohydrate and/or an antifoaming agent.

As used herein, the term "antifoaming agent" means a substance that completely or partially prevents the formation of foam and is used interchangeably with "foam-preventing agent". The antifoaming agent may include a medical antifoaming agent (Dow Corning®), without being limited thereto.

In the present invention, the medium composition for culturing *Clostridium botulinum* strains may contain a carbon source and a nitrogen source.

In the present invention, the carbon source may be a monosaccharide (e.g., glucose, fructose, etc.), a disaccharide (e.g., maltose, sucrose, etc.), an oligosaccharide, a polysaccharide (e.g., dextrin, cyclodextrin, starch, etc.), or a sugar alcohol (e.g., xylitol, sorbitol, erythritol, etc.), preferably D-(+)-glucose, without being limited thereto.

In the present invention, the content of the carbon source may be 0.1 to 1.5 w/v% (1 to 15 g/L), preferably 0.25 to 1.5 w/v% (2.5 to 15 g/L), preferably 1.0 w/v% (10 g/L), without being limited thereto.

As used herein, the term "nitrogen source" means an organic or inorganic substance containing nitrogen atoms essential for the synthesis of proteins, purines, pyrimidines, and polysaccharide chitin in the growth of strains. The nitrogen source is preferably yeast extract, without being limited thereto.

In the present invention, the nitrogen source may be yeast extract, polypeptone, tryptone, malt extract, corn steep liquor, or soytone, preferably yeast extract, without being limited thereto.

In the present invention, the content of the nitrogen source may be 0.5 to 2.0 w/v% (5 to 20 g/L), preferably 0.5 to 1.0 w/v% (5 to 10 g/L), preferably 1.0 w/v% (10 g/L), without being limited thereto.

In another aspect, the present invention relates to a method for producing botulinum toxin, comprising steps of: (a) producing botulinum toxin by culturing *Clostridium botulinum* using the medium composition; and (b) recovering the produced botulinum toxin.

In the present invention, the culturing may be performed under anaerobic conditions, the culturing may be performed under temperature conditions of 33°C to 37°C, the culturing may be performed for 20 to 48 hours, and the toxin may be selected from the group consisting of botulinum toxins A, B, C, D, E, F and G.

Hereinafter, the present invention will be described in more detail through examples. These examples are intended only to illustrate the present invention, and it will be obvious to those skilled in the art that the scope of the present invention is not to be construed as being limited by these examples.

## Example 1: Selection of plant peptones

### 1-1: Composition of CYG medium used for culture (control)

CYG medium (casein, yeast extract and glucose) for culturing *Clostridium botulinum* was prepared by dissolving 2% casein hydrolysate [20 g/L], 1% yeast extract [10 g/L], and 1% glucose [10 g/L] in 90 mL of distilled water, adjusting the final volume to 100 mL, and dispensing 10-mL aliquots of the prepared medium into 15-mL culture tubes. Thereafter, the medium was autoclaved at 121°C for 30 minutes and then kept under anaerobic conditions in an anaerobic incubator.

### 1-2: Compositions of APF media used for culture

As shown in Tables 1 and 2 below, 9 types of 2% plant peptone candidates (Sigma™ C8160-500G, Millipore™ 96174, Millipore™ 93492-500G-F, Sigma-Aldrich™ G5004-500G, Millipore™ 38143-500G, Organotechnie™ 19425, Sigma-Aldrich™ B4032-500G, Sigma-Aldrich™ 17218-500G, and Merck™ 1.05391.0500) [10 g/L] were each added to 1% yeast extract [10 g/L] and 1% dextrin or glucose [10 g/L], and then dissolved in 90 mL of water for injection. Then, the final volume was adjusted to 100 mL, and the pH of each medium was adjusted to 7.3. Then, 10-mL aliquots of the prepared media were dispensed into 15-mL culture tubes, respectively. Thereafter, the media were autoclaved at 121°C for 30 minutes and kept under anaerobic conditions in an anaerobic incubator.

[Table 1]

| Category | Medium (g/L) | | 1 | 2 | 3 | 4 | 5 | 6 | 7 | 8 | 9 |
|---|---|---|---|---|---|---|---|---|---|---|---|
| Carbon source | Dextrin | | 10 | 10 | 10 | 10 | 10 | 10 | 10 | 10 | 10 |
| | Glucose | | | | | | | | | | |
| Plant-derived components | Corn steep | Corn steep solid | 10 | | | | | | | | |
| | Pea | Peptone from pea | | 10 | | | | | | | |
| | Wheat glu-ten | Wheat peptone | | | 10 | | | | | | |
| | | Gluten from wheat | | | | 10 | | | | | |
| | Potato | Peotone from potatoes No. 2 | | | | | 10 | | | | |
| | | Potato peptone E210 | | | | | | 10 | | | |
| | Banana | Banana powder | | | | | | | 10 | | |
| | Tomato | Tomato juice | | | | | | | | 10 | |
| | Malt | Malt extract | | | | | | | | | 10 |
| Nitrogen source | Yeast extract | | 10 | 10 | 10 | 10 | 10 | 10 | 10 | 10 | 10 |

[Table 2]

| Category | Medium (g/L) | | 10 | 11 | 12 | 13 | 14 | 15 | 16 | 17 | 18 |
|---|---|---|---|---|---|---|---|---|---|---|---|
| Carbon source | Dextrin | | 10 | 10 | 10 | 10 | 10 | 10 | 10 | 10 | 10 |
| | Glucose | | | | | | | | | | |
| Plant-derived components | Corn steep | Corn steep solid | 10 | | | | | | | | |
| | Pea | Peptone from pea | | 10 | | | | | | | |
| | Wheat gluten | Wheat peptone | | | 10 | | | | | | |
| | | Gluten from wheat | | | | 10 | | | | | |
| | Potato | Peotone from | | | | | 10 | | | | |
| | | potatoes No. 2 | | | | | | | | | |
| | | Potato peptone E210 | | | | | | 10 | | | |
| | Banana | Banana powder | | | | | | | 10 | | |
| | Tomato | Tomato juice | | | | | | | | 10 | |
| | Malt | Malt extract | | | | | | | | | 10 |
| Nitrogen source | Yeast extract | | 10 | 10 | 10 | 10 | 10 | 10 | 10 | 10 | 10 |

### 1-3: Culture of *Clostridium botulinum* strain

Culture tubes containing medium compositions 1 to 18 shown in Tables 1 and 2 above were inoculated with 20 μL of *Clostridium botulinum* (Korea Center for Disease Control and Prevention, control No. 4-029-CBB-MF-2020001), and samples were taken while the strain was stationary-cultured under anaerobic conditions at 35°C±1°C for 20, 24, 28, and

48 hours. Thereafter, the growth rate of the strain was determined by measuring the $OD_{540nm}$ value of the culture sampled at each time using a UV spectrophotometer, and the culture medium was used as a blank. Based on the measured $OD_{540nm}$ value, plant peptone candidates for the medium were selected by comparison with the control.

**1-4: Strain growth rate**

Among the plant peptone candidates, corn extract solid, banana powder, and gluten from wheat were excluded from the plant peptone candidates because they formed precipitates after sterilization of the media, making it difficult to perform the filtration process and accurately measure the $OD_{540nm}$ value. The medium compositions containing the plant peptone candidates are shown in Table 3 below.

[Table 3]

| Medium (g/L) | | 1 | 2 | 3 | 4 | 5 | 6 | 7 | 8 | 9 | 10 | 11 | 12 |
|---|---|---|---|---|---|---|---|---|---|---|---|---|---|
| Dextrin | | 10 | 10 | 10 | 10 | 10 | 10 | | | | | | |
| Glucose | | | | | | | | 10 | 10 | 10 | 10 | 10 | 10 |
| Pea | Peptone from pea | 10 | | | | | | 10 | | | | | |
| Wheat gluten | Wheat peptone | | 10 | | | | | | 10 | | | | |
| Potato | Peptone from potatoes No. 2 | | | 10 | | | | | | 10 | | | |
| | Potato peptone E210 | | | | 10 | | | | | | 10 | | |
| Tomato | Tomato juice | | | | | 10 | | | | | | 10 | |
| Malt | Malt extract | | | | | | 10 | | | | | | 10 |
| Yeast extract | | 10 | 10 | 10 | 10 | 10 | 10 | 10 | 10 | 10 | 10 | 10 | 10 |

The $OD_{540nm}$ values of cultures sampled at each time point for medium compositions 1 to 12 shown in Table 3 above and two control media (CYG media) were measured (Table 4), and the graphs of the $OD_{540nm}$ values of the carbon source (dextrin or glucose) of each medium over time were compared (FIG. 4).

[Table 4]

| **Medium** | **$OD_{540nm}$ value over time** | | | |
|---|---|---|---|---|
| | **20hr** | **24hr** | **28hr** | **48hr** |
| **1** | **1.8730** | **2.3500** | **3.2304** | **3.2722** |
| **2** | **1.8599** | **2.1685** | **2.5121** | **4.0037** |
| **3** | **1.4141** | **1.5862** | **2.0204** | **2.4978** |
| **4** | **1.4811** | **2.0280** | **2.3678** | **2.6301** |
| **5** | **1.0544** | **1.1912** | **1.3145** | **0.8669** |
| **6** | **1.1858** | **1.6250** | **1.8331** | **1.4966** |
| **7** | **2.2260** | **3.0544** | **3.7671** | **4.0443** |
| **8** | **2.3389** | **3.2501** | **3.6515** | **2.6177** |
| **9** | **1.5869** | **1.9006** | **1.9675** | **2.0508** |
| **10** | **1.2805** | **1.2364** | **1.1067** | **1.8789** |
| **11** | **0.9147** | **1.0896** | **1.1602** | **1.3564** |
| **12** | **1.0836** | **1.4444** | **1.6244** | **0.5030** |
| **Control 1** | **2.0969** | **2.8093** | **3.0028** | **2.3717** |
| **Control 2** | **1.1 039** | **1.6976** | **2.0191** | **2.2898** |

As a result, as shown in Table 4 above and FIG. 4, it was confirmed that the strain showed high growth rates

commonly in media 1, 2, 3, 7, 8 and 9 containing components derived from pea, wheat, and potato.

In addition, it was confirmed that, even when the same plant peptones derived from pea, wheat, and potato were used, the strain showed higher growth rates in media 7, 8 and 9 containing glucose as a carbon source than in media 1, 2 and 3 containing dextrin. In particular, it was confirmed that, in the case of wheat-derived components, the strain tended to grow even after 48 hours after inoculation in medium 2 containing dextrin (FIG. 4). This growth trend may unnecessarily delay the production time of the stock solution in the future, suggesting that using glucose as a carbon source for the medium is suitable.

**1-5: Botulinum toxin concentration**

The amounts of toxin protein in media 1, 2, 3, 7, 8, and 9, which showed high growth rates of the strain in Examples 1-4, were measured. Since previous studies recover toxins during the death phase, toxin concentrations were measured by the ELISA method for the quantitative analysis of botulinum toxin type A culture medium at 28 and 48 hours, which are the latter parts of the culture, and the results are shown in Table 5 below (ICH guideline, Q2(R1)).

Antitoxin was diluted and dispensed into each well of a 96-well plate, which was then coated for 16 hours or more. The contents of the plate were removed, each well was blocked with blocking solution, and then the plate was washed three times with wash buffer. The standard and the culture were diluted and dispensed into each well in triplicate, and the plate was incubated and then washed three times with wash buffer. Primary antibody dilution solution was dispensed into each well, and the plate was incubated and then washed three times with wash buffer. Substrate solution was dispensed into each well and incubated, and then stop solution was dispensed into each well to stop the reaction. Absorbance was measured at wavelengths of 450 to 540 nm.

[Table 5]

| Medium | Plant-/animal-derived component | Carbon source | Toxin concentration (ng/mL) | |
|---|---|---|---|---|
| | | | 28 hr | 48 hr |
| Control (CYG) | Casein | Glucose | 17826.0 | 20007.7 |
| 1 | Pea | Dextrin | 11716.8 | 6676.7 |
| 2 | Wheat | | 4294.4 | 5703.5 |
| 3 | Potato | | 14732.2 | 9945.9 |
| 7 | Pea | Glucose | 20775.2 | 11772.9 |
| 8 | Wheat | | 5135.8 | 17415.1 |
| 9 | Potato | | 14440.7 | 16602.9 |

As a result, as shown in Table 5 above, the toxin expression levels were highest in the order of pea, potato, and wheat. In addition, it was confirmed that, when the same plant peptones were used, the toxin expression levels in the media containing glucose as the carbon source were higher than those in the medium containing dextrin, and that using glucose as the carbon source for the medium for producing toxin from the *Clostridium botulinum* strain was more suitable.

Based on the experimental results of Example 1, three plant peptones were selected as final plant peptone candidates: peptone from pea, wheat peptone, and potato peptone (peptone from potatoes No. 2).

**Example 2: Selection of proportion of plant peptones**

**2-1: Method for selection of proportion of plant peptones**

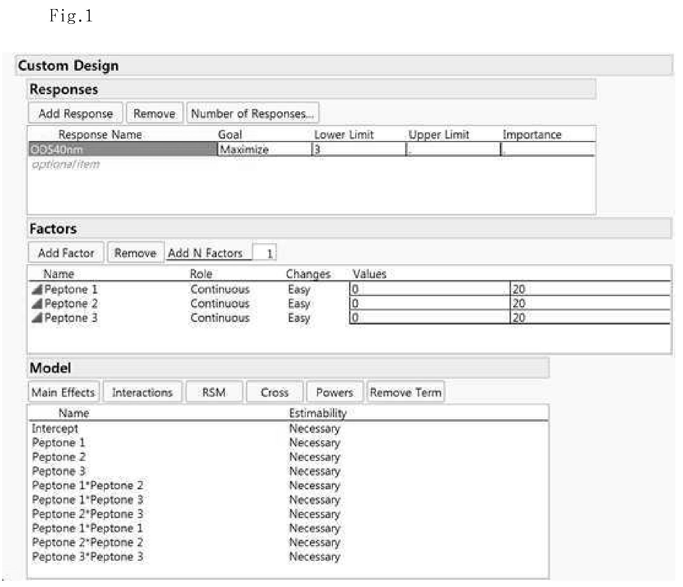

In order to select the proportion of the three final plant peptone candidates finally selected in Examples 1-4 and 1-5, a JMP custom design was used to set the $OD_{540nm}$ value as a response to 3 or higher and the proportion of the three plant peptones as factors to 2% or less. In addition, in the same manner as in Examples 1-1 and 1-2, the proportion of each of the carbon source and the yeast extract was fixed at 1% and the experiment was conducted in duplicate per medium condition (FIG. 1; Peptone 1: peptone from pea; Peptone 2: wheat peptone; Peptone 3: potato peptone).

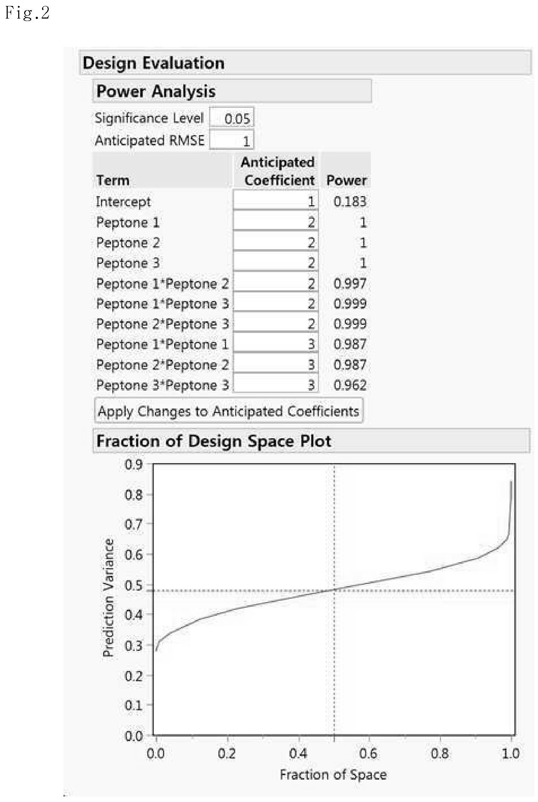

For design evaluation, power analysis was performed and the fraction of design space plot was examined (FIG. 2; Peptone 1: peptone from pea; Peptone 2: wheat peptone; Peptone 3: potato peptone).

The experimental design for $OD_{540nm}$ values according to the proportion of plant peptones obtained through custom design is shown in Table 6 below, and the toxin concentration after the completion of culture under each condition was measured and added to the analysis items (Peptone 1: peptone from pea; Peptone 2: wheat peptone; Peptone 3:

potato peptone).

[Table 6]

| | Peptone 1 | Peptone 2 | Peptone 3 | OD540nm |
|---|---|---|---|---|
| 1 | 10 | 20 | 0 | |
| 2 | 10 | 0 | 10 | |
| 3 | 20 | 10 | 0 | |
| 4 | 10 | 0 | 0 | |
| 5 | 10 | 10 | 20 | |
| 6 | 0 | 10 | 0 | |
| 7 | 0 | 0 | 0 | |
| 8 | 20 | 20 | 0 | |
| 9 | 0 | 20 | 20 | |
| 10 | 0 | 20 | 10 | |
| 11 | 20 | 20 | 20 | |
| 12 | 20 | 0 | 0 | |
| 13 | 20 | 0 | 20 | |
| 14 | 0 | 20 | 0 | |
| 15 | 20 | 10 | 10 | |
| 16 | 0 | 0 | 20 | |

**2-2: Compositions of APF media used for culture**

As shown in Table 7 below, to 1% glucose [10 g/L] as a carbon source and 1% yeast extract [10 g/L], 2% three plant peptones [10 g/L] were added. Each of the mixtures was dissolved in 90 mL of water for injection, and the final volume was adjusted to 100 mL. 10-mL aliquots of the prepared media were then dispensed into 15-mL culture tubes, respectively. Thereafter, the media were autoclaved at 121°C for 30 minutes and then kept under anaerobic conditions in an anaerobic incubator.

[Table 7]

| | Medium composition (g/L) | | | | | | | | | | | | | | | |
|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 |
| Carbon source | 10 | 10 | 10 | 10 | 10 | 10 | 10 | 10 | 10 | 10 | 10 | 10 | 10 | 10 | 10 | 10 |
| Yeast extract | 10 | 10 | 10 | 10 | 10 | 10 | 10 | 10 | 10 | 10 | 10 | 10 | 10 | 10 | 10 | 10 |
| Peptone from pea | 10 | 10 | 20 | 10 | 10 | 0 | 0 | 20 | 0 | 0 | 20 | 20 | 20 | 0 | 20 | 0 |
| Wheat peptone | 20 | 0 | 10 | 0 | 10 | 10 | 0 | 20 | 20 | 20 | 20 | 0 | 0 | 20 | 10 | 0 |
| Potato peptone | 0 | 10 | 0 | 0 | 20 | 0 | 0 | 0 | 20 | 10 | 20 | 0 | 20 | 0 | 10 | 20 |

**2-3: Culture of *Clostridium botulinum* strain**

Culture tubes containing medium compositions 1 to 16 shown in Table 7 above were inoculated with 20 μL of *Clostridium botulinum* (Korea Center for Disease Control and Prevention, control No. 4-029-CBB-MF-2020001), and samples were taken while the strain was stationary-cultured under anaerobic conditions at 35°C±1°C for 20, 24, 28, and 48 hours. Thereafter, the growth rate of the strain was determined by measuring the $OD_{540nm}$ value of the culture sampled at each time using a UV spectrophotometer, and the culture medium was used as a blank. Based on the measured $OD_{540nm}$ value, plant peptone candidates for the medium were selected by comparison with the control. Based on the measured $OD_{540nm}$ value, the proportion of addition of plant peptones for the medium was selected.

**2-4: Strain growth rate**

The $OD_{540nm}$ values of the cultures sampled at each time point for medium compositions 1 to 16 shown in Table 7

above were measured (Table 8), and the graphs of the $OD_{540nm}$ values over time for each medium were compared (FIG. 5).

[Table 8]

| Medium r | $OD_{540nm}$ value over time | | | |
|---|---|---|---|---|
| | 20hr | 24hr | 28hr | 48hr |
| 1 | 4.2407 | 6.0792 | 6.6703 | 7.6256 |
| 2 | 2.9021 | 3.1707 | 3.7849 | 4.5212 |
| 3 | 4.3292 | 5.5489 | 6.7432 | 5.1707 |
| 4 | 2.3007 | 2.6335 | 3.6120 | 3.9954 |
| 5 | 3.3414 | 4.4627 | 4.8113 | 4.5013 |
| 6 | 2.2590 | 2.8932 | 3.7034 | 3.1049 |
| 7 | 0.2857 | 0.1835 | 1.1849 | 0.6199 |
| 8 | 4.2728 | 6.7621 | 8.1035 | 4.7047 |
| 9 | 4.2576 | 4.2045 | 5.4406 | 5.8829 |
| 10 | 3.3709 | 3.7021 | 5.1411 | 4.3266 |
| 11 | 5.1641 | 5.6140 | 6.6381 | 2.2498 |
| 12 | 3.7988 | 4.7715 | 5.9342 | 4.2629 |
| 13 | 5.35.45 | 5.1057 | 6.5547 | 2.0944 |
| 14 | 2.8511 | 3.3602 | 3.7440 | 4.6341 |
| 15 | 4.8876 | 3.4032 | 5.8843 | 5.2539 |
| 16 | 2.2215 | 2.8578 | 2.7791 | 3.8910 |

**2-5: Analysis of results of Design of Experiment (DoE)**

For medium compositions 1 to 16 shown in Table 7 above, the $OD_{540nm}$ value at 24 hours, which is the primary seed culture time in previous studies on *Clostridium botulinum,* was used for analysis, and since the toxin is recovered during the death phase, the amount of toxin protein, the value measured by ELISA at 48 hours were used for the amount of toxin protein (Table 9). Based on these values, the results were statistically analyzed using JMP.

[Table 9]

| | Pea | Wheat | Potato | OD at 24hr | Toxin at 48hr |
|---|---|---|---|---|---|
| 1 | 10 | 20 | 0 | 6.0792 | 42041.2 |
| 2 | 10 | 0 | 10 | 3.1707 | 56296.2 |
| 3 | 20 | 10 | 0 | 5.54885 | 64887.3 |
| 4 | 10 | 0 | 0 | 2.6335 | 44518.3 |
| 5 | 10 | 10 | 20 | 4.46265 | 44377.2 |
| 6 | 0 | 10 | 0 | 2.89315 | 35791.2 |
| 7 | 0 | 0 | 0 | 0.18351 | 16783 |
| 8 | 20 | 20 | 0 | 6.7621 | 56713.4 |
| 9 | 0 | 20 | 20 | 4.2045 | 19848.6 |
| 10 | 0 | 20 | 10 | 3.7021 | 34152.4 |
| 11 | 20 | 20 | 20 | 5.61395 | 34592.6 |
| 12 | 20 | 0 | 0 | 4.7715 | 50099.6 |
| 13 | 20 | 0 | 20 | 5.10565 | 49023.4 |
| 14 | 0 | 20 | 0 | 3.36015 | 20371.4 |
| 15 | 20 | 10 | 10 | 3.4032 | 35153.9 |
| 16 | 0 | 0 | 20 | 2.8578 | 25383.1 |

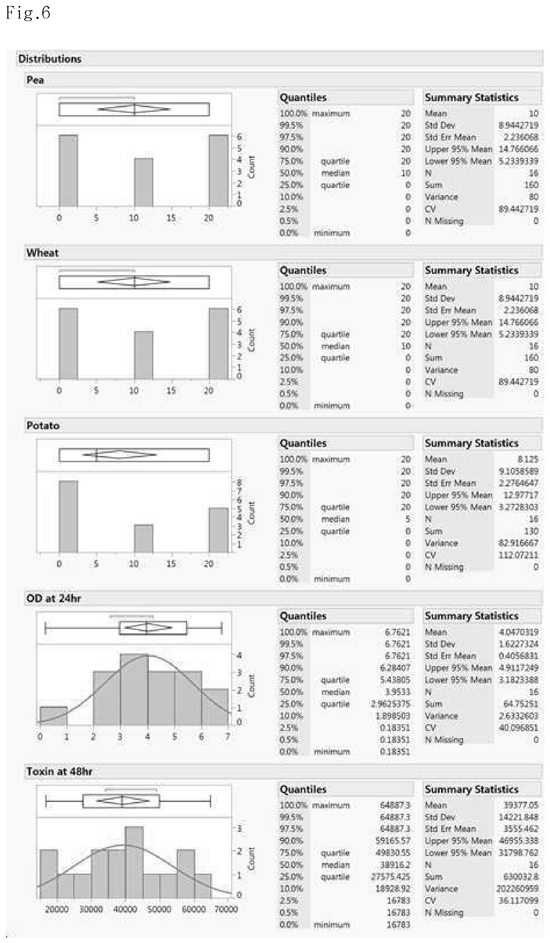

Through distribution analysis of the results for each condition as shown in Table 9 above, it was confirmed that the experimental design was D-optimal and that the experimental results were generally evenly distributed (FIG. 6).

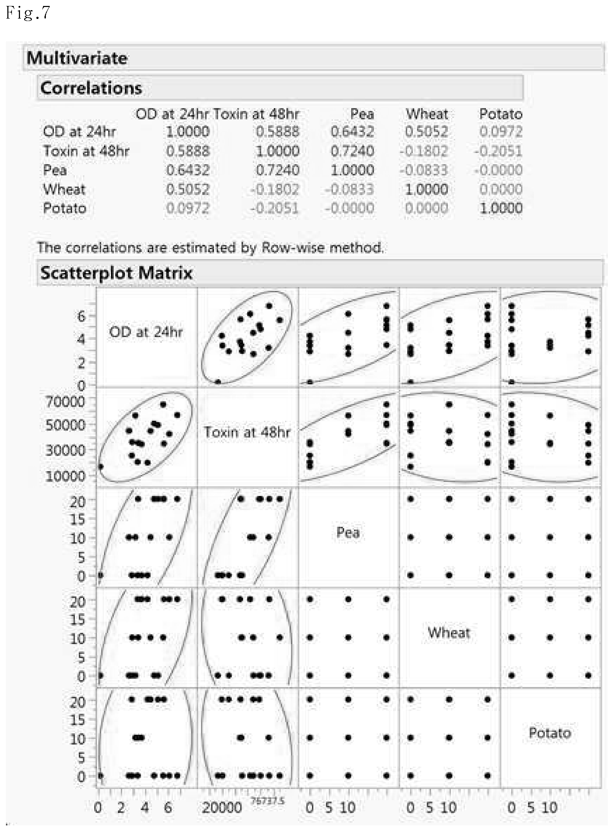

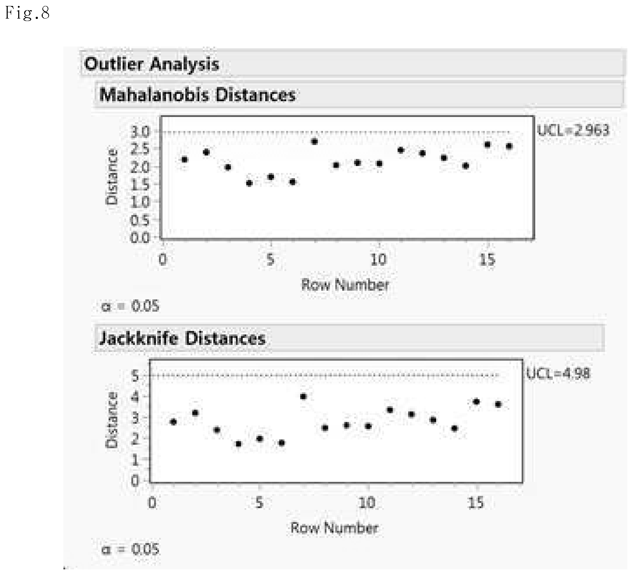

According to scatterplot matrix analysis, all correlation values were 0.75 or lower, indicating a weak correlation. The R values between the X variables (pea, wheat, and potato) were close to 0, indicating a very weak correlation, so there was no interaction. It was confirmed that there was a weak positive correlation between the Y variables ($OD_{540nm}$, and toxin concentration), between pea and the Y variable, and between wheat and the $OD_{540nm}$ value (FIG. 7). Outlier analysis confirmed that there were no outliers in the experimental results (FIG. 8).

**2-6: Analysis of $OD_{540nm}$ value depending on type of plant peptone**

A fit model was established based on the results of analysis of $OD_{540nm}$ value depending on the type of plant peptone. The model was established by the effect leverage method using least squares, and the X variables were analyzed using the response surface method. To select the optimal model, the R-squared value and RMSE value were considered together, and after checking effect summary and the p-value of effect test, terms that were not significant were deleted.

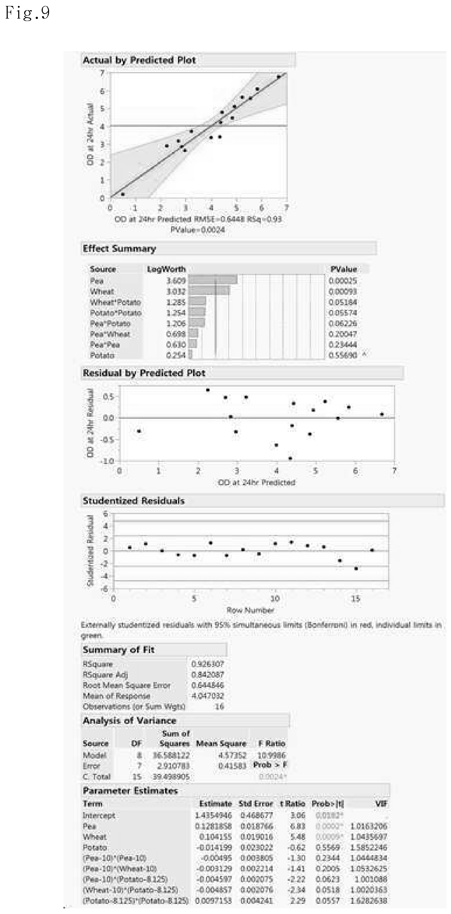

As a result of checking the VIF value of the optimal established model, it was confirmed that the value did not exceed 10 and was at level 1, suggesting that there was no problem with the model. In residual analysis, it was confirmed that points were evenly distributed up and down and all studentized residuals results were included in the 95% confidence interval (FIG. 9).

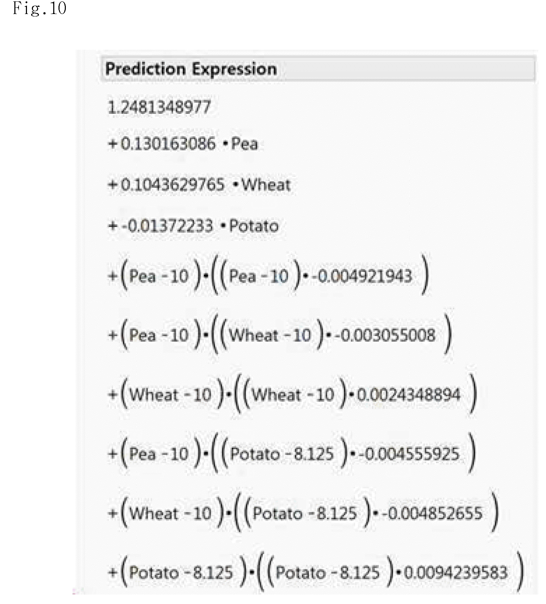

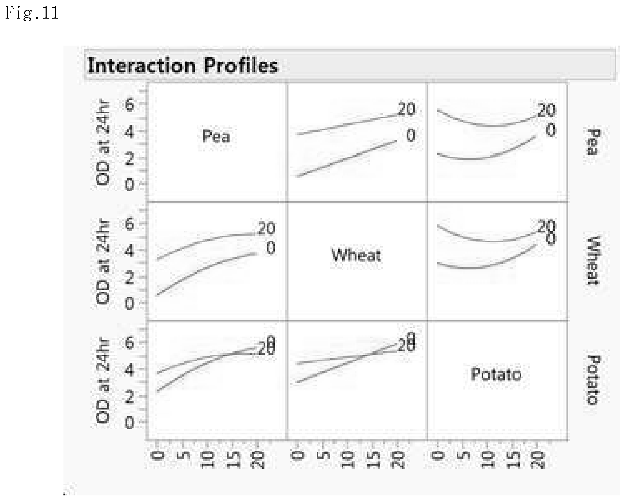

As a result of identifying the factors that significantly affect the model through the effect test results, it was found that pea and wheat had an effect on the model (Table 10). A prediction formula for $OD_{540nm}$ was obtained using this model (FIG. 10). As a result of examining the main effects and interaction profiles, it was confirmed that there were interactions between pea and potato, and between wheat and potato, but there were no interactions between the other factors (FIG. 11).

[Table 10]

Effect Tests

| Source | Nparm | DF | Sum of Squares | F Ratio | Prob > F |
|---|---|---|---|---|---|
| Pea | 1 | 1 | 19.476398 | 42.6410 | 0.0006* |
| Wheat | 1 | 1 | 12.520622 | 27.4122 | 0.0019* |
| Potato | 1 | 1 | 0.147585 | 0.3231 | 0.5904 |
| Pea*Pea | 1 | 1 | 0.695719 | 1.5232 | 0.2633 |
| Pea*Wheat | 1 | 1 | 0.789833 | 1.7292 | 0.2365 |
| Wheat*Wheat | 1 | 1 | 0.170263 | 0.3728 | 0.5639 |
| Pea*Potato | 1 | 1 | 2.003438 | 4.3863 | 0.0811 |
| Wheat*Potato | 1 | 1 | 2.272906 | 4.9762 | 0.0672 |
| Potato*Potato | 1 | 1 | 2.029966 | 4.4443 | 0.0796 |

**2-7: Analysis of toxin expression level depending on type of plant peptone**

A fit model was established based on the results of ELISA. The model was established by the effect leverage method using least squares, and the X variables were analyzed using the response surface method. To select the optimal model, the R-squared value and RMSE value were considered together, and after checking effect summary and the p-value of effect test, terms that were not significant were deleted.

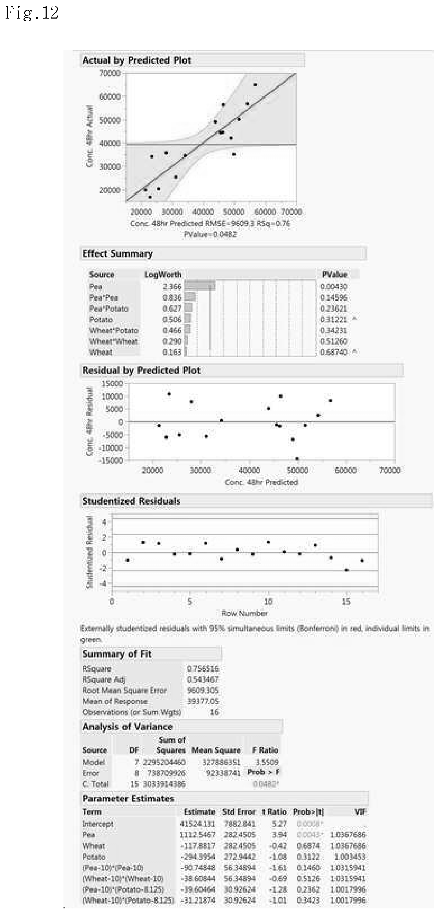

As a result of checking the VIF value of the optimal established model, it was confirmed that the value did not exceed 10 and was at level 1, suggesting that there was no problem with the model. In residual analysis, it was confirmed that points were evenly distributed up and down and all studentized residuals results were included in the 95% confidence interval (FIG. 12).

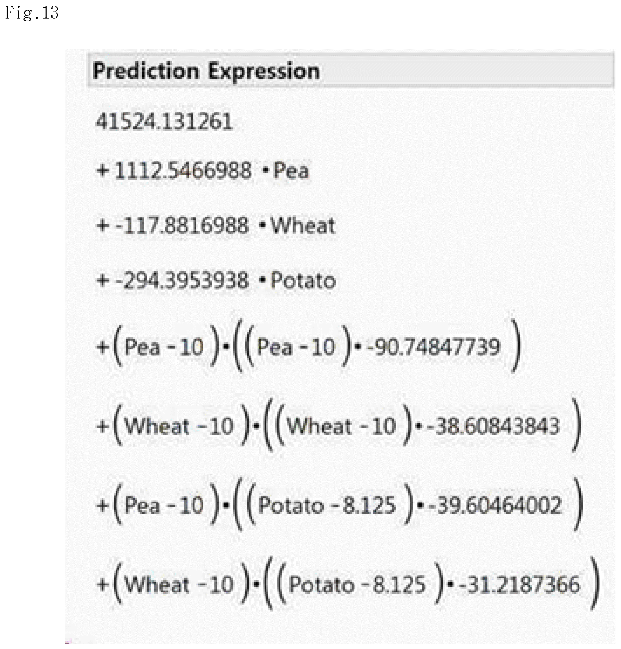

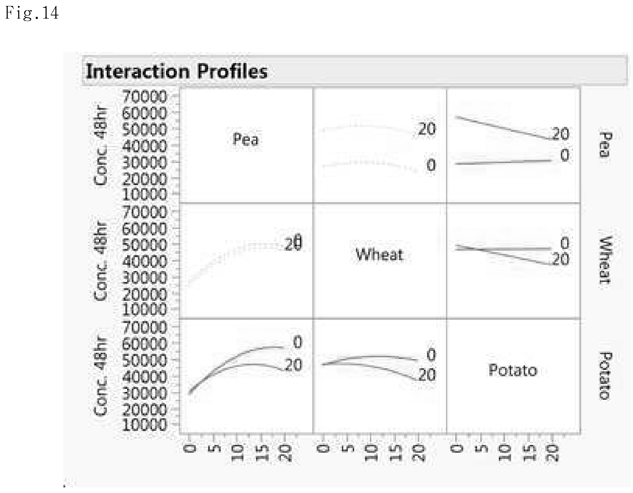

As a result of identifying the factors that significantly affect the model through the effect test results, it was found that pea had an effect on the model (Table 11). A prediction formula for $OD_{540nm}$ was obtained using this model (FIG. 13). As a result of examining the main effects and interaction profiles, it was confirmed that there were interactions between pea and potato, and between wheat and potato, but there were no interactions between the other factors (FIG. 14).

[Table 11]

Effect Tests

| Source | Nparm | DF | Sum of Squares | F Ratio | Prob > F |
|---|---|---|---|---|---|
| Pea | 1 | 1 | 1432636114 | 15.5150 | 0.0043* |
| Wheat | 1 | 1 | 16083929.8 | 0.1742 | 0.6874 |
| Potato | 1 | 1 | 107423198 | 1.1634 | 0.3122 |
| Pea*Pea | 1 | 1 | 239492040 | 2.5936 | 0.1460 |
| Wheat*Wheat | 1 | 1 | 43348778.2 | 0.4695 | 0.5126 |
| Pea*Potato | 1 | 1 | 151433498 | 1.6400 | 0.2362 |
| Wheat*Potato | 1 | 1 | 94093681.4 | 1.0190 | 0.3423 |

**2-8: Robust optimization**

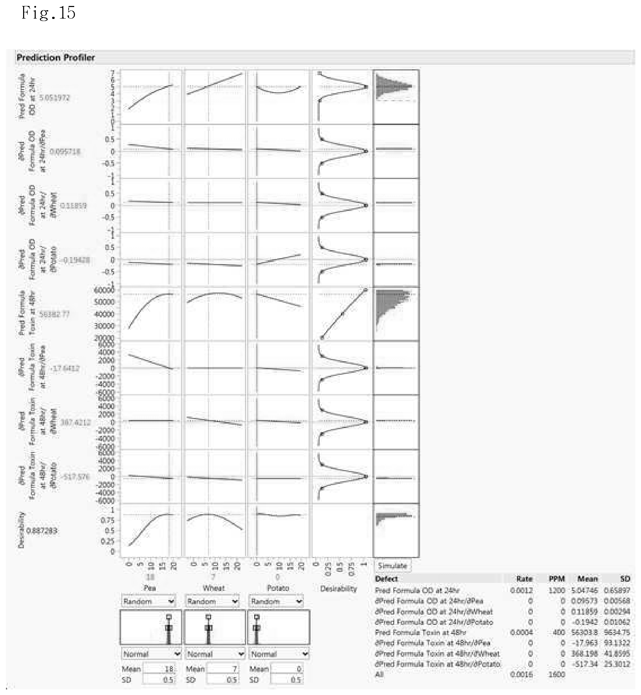

Based on the prediction formula established for each response value, robust optimization conditions were identified using a profiler to determine the medium conditions that can obtain the appropriate $OD_{540nm}$ and the maximum toxin concentration. In the actual culture process, for the $OD_{540nm}$ value, a cell state is selected that can continue to grow after passage while maintaining the exponential phase. Therefore, as the minimum cell concentration for passage in the exponential phase was at an $OD_{540nm}$ value of 3 to 7, the appropriate conditions were explored by setting the $OD_{540nm}$ value to a match target of 5 based on the established model formula. For the toxin concentration, the ELISA value was set to maximum value to find the composition that produces the highest concentration of toxin. As a result of performing an analysis to maximize desirability for the two response values, the use of only pea and wheat was determined, and potato

was excluded from subsequent analyses (FIG. 15).

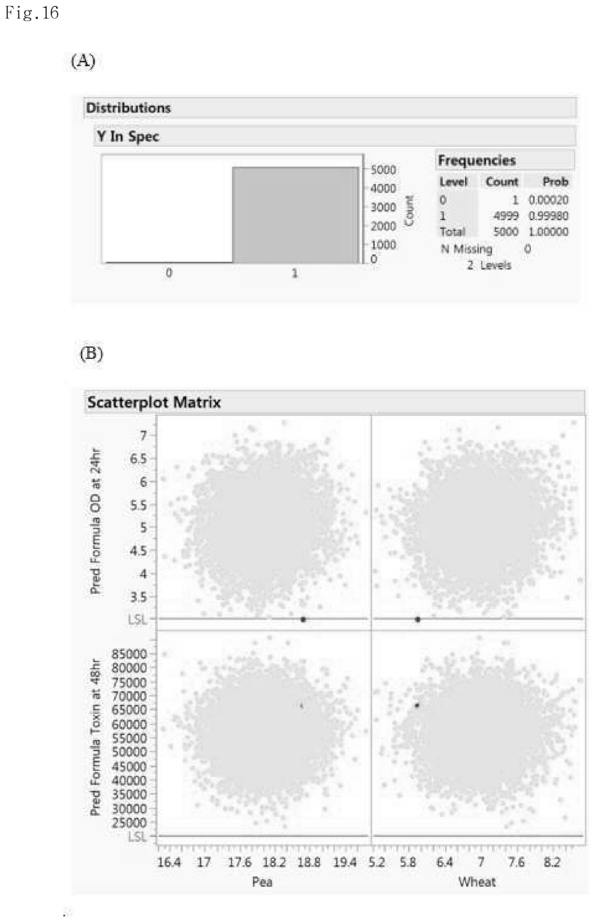

After performing 5,000 simulations as a function of the random variation in the factors and model noise using the simulator function (FIG. 16a), the simulation results were presented through edge of failure analysis. It was confirmed that all response values fell within the spec limit except for one case out of 5,000 (FIG. 16b).

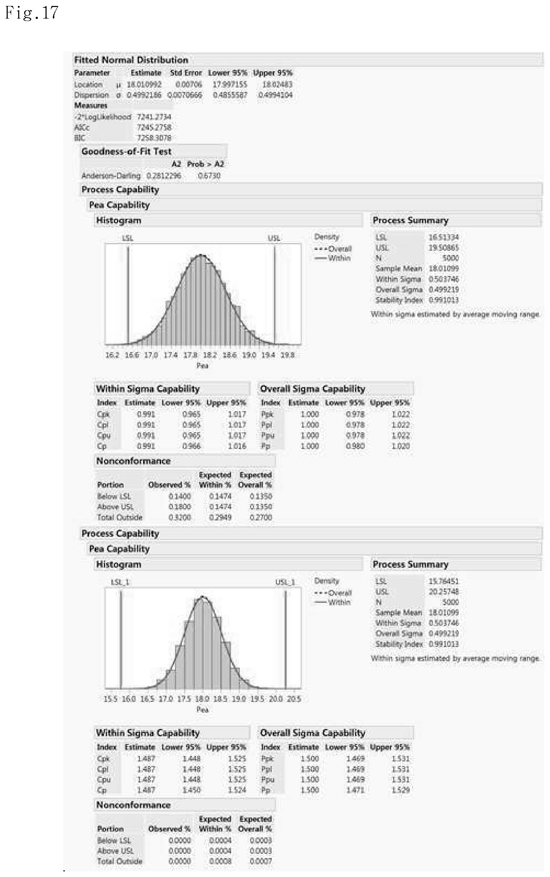

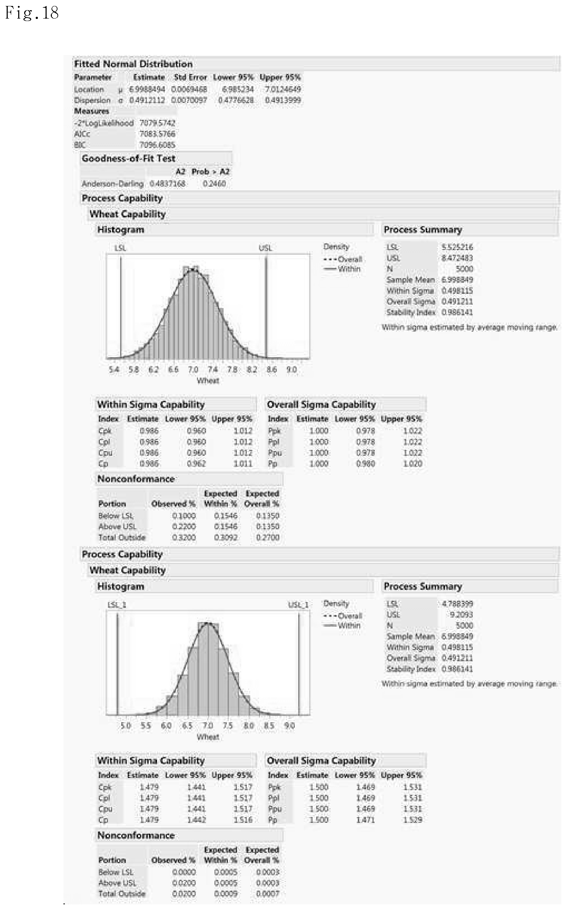

To obtain results under appropriate conditions, the acceptable ranges for each component were determined. The range corresponding to 3σ was set as NORs (a range representing 99.7% of the actual result distribution), and the range corresponding to 4.5σ was set as PARs (FIGS. 17 and 18). Based on this, the determined acceptable ranges for each component are shown in Table 12 below.

[Table 12]

| Component (g/L) | Set point | NORs (3σ) | PARs (4.5σ) |
|---|---|---|---|
| Peptone from pea | 18 g/L | 16.5 to 19.5 | 15.8 to 20.3 |
| Wheat peptone | 7 g/L | 5.5 to 8.5 | 4.8 to 9.2 |

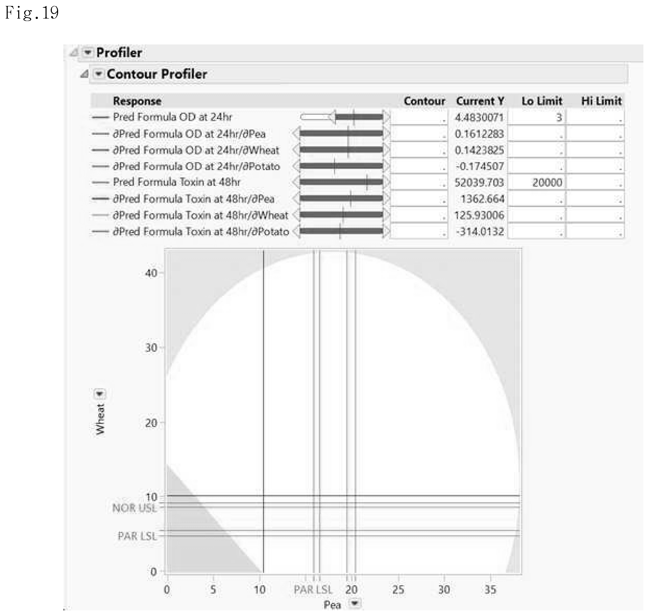

For the convenience of the operator upon application to the actual process, the set point was 18 g/L for peptone from pea and 7 g/L for wheat peptone. As a result of plotting NORs (3σ, red line) and PARs (4.5σ, blue line) on a contour plot to find the range satisfying the model, it was confirmed that NORs and PARs did not exceed the white range satisfying the process conditions, suggesting that the NORs and PARs set by this test and statistical analysis were suitable (FIG. 19).

**Example 3: Comparative measurement of absorbance and toxin expression level for APF media**

**3-1: Compositions of media used for culture**

To APF medium for culturing *Clostridium botulinum*, peptone from pea and wheat peptone were added in amounts selected in Examples 2-8, and D-(+)-glucose as a carbon source and yeast extract as a nitrogen source were added in amounts identical to those in Examples 1-2.

Meanwhile, to determine the effect of L-cysteine hydrochloride monohydrate or antifoaming agent emulsion on the APF medium, 0.2 g/L L-cysteine hydrochloride monohydrate and/or 0.24 g/L medical antifoam C emulsion (Dow Corning®) was added to the APF medium. In addition, to determine the effect of soy peptone on the APF medium, 18 g/L soy peptone was added instead of the pea peptone and/or wheat peptone of the APF medium, and additionally, 0.2 g/L L-cysteine hydrochloride monohydrate and 0.24 g/L medical antifoam C emulsion (Dow Corning®) were added.

10-mL aliquots of the media were dispensed into 15-mL culture tubes, respectively. Thereafter, the media were autoclaved at 121°C for 30 minutes and then kept under anaerobic conditions in an anaerobic incubator. The APF media for culturing *Clostridium botulinum* and the specific compositions of the media are shown in Table 13 below.

[Table 13]

| Component (g/L) | APF medium | Comp. Example 1 | Comp. Example 2 | Comp. Example 3 | Comp. Example 4 | Comp. Example 5 | Comp. Example 6 | Comp. Example 7 |
|---|---|---|---|---|---|---|---|---|
| Peptone from pea | 10 | 10 | 10 | 10 | - | - | - | - |
| Wheat peptone | 7 | 7 | 7 | 7 | - | 7 | 7 | 20 |
| Yeast extract | 10 | 10 | 10 | 10 | 10 | 10 | 10 | 20 |
| D-(+)-glucose | 10 | 10 | 10 | 10 | 10 | 10 | 10 | 10 |
| Soy peptone | - | - | - | - | 10 | 10 | 10 | |
| L-cysteine HCl | - | 0.2 | - | 0.2 | - | - | 0.2 | 0.2 |
| Antifoam C emulsion | - | - | 0.24 | 0.24 | - | - | 0.24 | 0.24 |

### 3-2: Culture of *Clostridium botulinum* strain

Culture tubes containing the media shown in Table 13 were inoculated with 20 μL of *Clostridium botulinum* (Korea Center for Disease Control and Prevention, control No. 4-029-CBB-MF-2020001), and samples were taken while the strain was stationary-cultured under anaerobic conditions at 35°C±1°C for 20, 24, 28, and 48 hours. Thereafter, the growth rate of the strain was determined by measuring the $OD_{540nm}$ value of the culture sampled at each time using a UV spectrophotometer, and the culture medium was used as a blank.

### 3-3: Strain growth rate and toxin expression level

The $OD_{540nm}$ value of the cultures sampled at each time for the media shown in Table 13 above and the toxin expression levels after 48 hours of culture were measured (Tables 14 and 15, and FIGS. 20 and 21).

[Table 14]

| | APF medium | Comp. Example 1 | Comp. Example 2 | Comp. Example 3 | Comp. Example 4 | Comp. Example 5 | Comp. Example 6 | Comp. Example 7 |
|---|---|---|---|---|---|---|---|---|
| OD at 20 hr | 4.0474 | 3.7144 | 1.7721 | 3.9557 | 2.2205 | 2.8330 | 1.7535 | 3.7871 |
| OD at 24 hr | 5.9563 | 5.3636 | 2.0319 | 5.6034 | 2.9668 | 3.4903 | 2.7539 | 5.4549 |
| OD at 28 hr | 7.1021 | 6.2576 | 1.7567 | 7.1667 | 3.5252 | 4.2801 | 3.2109 | 6.7739 |
| OD at 48 hr | 4.5636 | 5.3044 | 1.5032 | 4.5062 | 4.4182 | 5.3889 | 3.7297 | 7.8900 |
| Toxin (μg) at 48 hr | 588.8 | 452.1 | 137.9 | 501.1 | 75.2 | 76.4 | 30.1 | 263.7 |
| Toxin expression (%) compared to control | - | -23.2 | -76.6 | -14.9 | -87.2 | -87.0 | -94.9 | -55.2 |

[Table 15]

| | | Measured value (ng/mL) | Average value | Culture (mL) | Toxin amount (μg) |
|---|---|---|---|---|---|
| APF medium | Tube 1 | 46894.1 | 58880.0 | 10 | 588.8 |
| | Tube 2 | 70867.4 | | | |
| Comparative Example 1 | Tube 1 | 51505.6 | 45208.9 | 10 | 452.1 |
| | Tube 2 | 38912.1 | | | |
| Comparative Example 2 | Tube 1 | 13052.2 | 13972.6 | 10 | 137.9 |
| | Tube 2 | 14533.0 | | | |
| Comparative Example 3 | Tube 1 | 57599.9 | 50110.8 | 10 | 501.1 |
| | Tube 2 | 42621.6 | | | |
| Comparative Example 4 | Tube 1 | 6196.3 | 7522.7 | 10 | 75.2 |
| | Tube 2 | 8849.1 | | | |
| Comparative Example 5 | Tube 1 | 7051.5 | 7644.9 | 10 | 76.4 |
| | Tube 2 | 8238.4 | | | |
| Comparative Example 6 | Tube 1 | 2696.2 | 3012.2 | 10 | 30.1 |
| | Tube 2 | 3328.3 | | | |
| Comparative Example 7 | Tube 1 | 29852.4 | 26366.1 | 10 | 263.7 |

As a result, as shown in Table 14 above and FIGS. 20 and 21, it was confirmed that, when *Clostridium botulinum*

was cultured in the APF medium of the present invention, the growth of APF was best at 20 to 28 hours, and the level of toxin expression was highest at 48 hours after the growth of the strain.

Meanwhile, it was confirmed that, in the cases of Comparative Example 1, in which only L-cysteine HCl monohydrate was added to the APF medium of the present invention, Comparative Example 2, in which only medical antifoam C emulsion was added to the APF medium of the present invention, and Comparative Example 3, in which L-cysteine HCl monohydrate and medical antifoam C emulsion were added to the APF medium of the present invention, strain growth was lower than that in the APF medium of the present invention, and the levels of toxin expression decreased by 23.2%, 76.6%, and 14.9%, respectively. Accordingly, it can be confirmed that, even if L-cysteine HCl monohydrate and/or medical antifoam C emulsion are added to the APF medium composition of the present invention, strain growth and toxin expression in the APF medium composition of the present invention are excellent.

Meanwhile, it was confirmed that, in the cases of Comparative Example 4, in which soy peptone in place of peptone from pea and wheat peptone was added to the APF medium of the present invention, Comparative Example 5, in which soy peptone in place of peptone from pea was added to the APF medium of the present invention, Comparative Example 6, in which soy peptone in place of peptone from pea was added to the APF medium of the present invention and additionally L-cysteine HCl monohydrate and medical antifoam C emulsion were added, and Comparative Example 7, which has the same composition as that in Korean Patent Application Publication No. 10-2022-0140696, strain growth was lower than that in the APF medium of the present invention, and the levels of toxin expression decreased by 87.2%, 87.0%, 94.9%, and 55.2%, respectively. Accordingly, considering that the time required to reach the death phase where the toxin can be recovered is expected to be 72 hours or more, it can be confirmed that strain growth and toxin expression in the APF medium composition of the present invention are excellent.

## Claims

1. A medium composition for culturing *Clostridium botulinum* strains, comprising: plant peptones, including wheat peptone and peptone from pea; D-(+)-glucose; and yeast extract.

2. The medium composition according to claim 1, not comprising an animal-derived component.

3. The medium composition according to claim 2, wherein the animal-derived component is casein hydrolysate.

4. The medium composition according to claim 1, wherein a content of the wheat peptone is 0.1 to 4.0 w/v%.

5. The medium composition according to claim 1, wherein a content of the peptone from pea is 0.1 to 3.5 w/v%.

6. The medium composition according to claim 1, wherein contents of the D-(+)-glucose and the yeast extract are each 0.005 to 2 w/v%.

7. A method for producing botulinum toxin, comprising steps of:

   (a) producing botulinum toxin by culturing *Clostridium botulinum* using the medium composition according to any one of claims 1 to 6; and
   (b) recovering the produced botulinum toxin.

8. The method according to claim 7, wherein the culturing is performed under anaerobic conditions.

9. The method according to claim 7, wherein the culturing is performed under temperature conditions of 33°C to 37°C.

10. The method according to claim 7, wherein the culturing is performed for 20 to 48 hours.

11. The method according to claim 7, wherein the toxin is selected from the group consisting of botulinum toxins A, B, C, D, E, F and G.

Fig.1

Custom Design
Responses
Add Response
Remove
Number of Responses...
Response Name
Goal
Lower Limit
Upper Limit
Importance
OD540nm
Maximize
3
optional item
Factors
Add Factor
Remove
Add N Factors
1
Name
Role
Changes
Values
Peptone 1
Continuous
Easy
0
20
Peptone 2
Continuous
Easy
0
20
Peptone 3
Continuous
Easy
0
20
Model
Main Effects
Interactions
RSM
Cross
Powers
Remove Term
Name
Estimability
Intercept
Necessary
Peptone 1
Necessary
Peptone 2
Necessary
Peptone 3
Necessary
Peptone 1*Peptone 2
Necessary
Peptone 1*Peptone 3
Necessary
Peptone 2*Peptone 3
Necessary
Peptone 1*Peptone 1
Necessary
Peptone 2*Peptone 2
Necessary
Peptone 3*Peptone 3
Necessary

Fig.2

Design Evaluation
Power Analysis
Significance Level 0.05
Anticipated RMSE 1
Term
Anticipated Coefficient
Power
Intercept 1 0.183
Peptone 1 2 1
Peptone 2 2 1
Peptone 3 2 1
Peptone 1*Peptone 2 2 0.997
Peptone 1*Peptone 3 2 0.999
Peptone 2*Peptone 3 2 0.999
Peptone 1*Peptone 1 3 0.987
Peptone 2*Peptone 2 3 0.987
Peptone 3*Peptone 3 3 0.962
Apply Changes to Anticipated Coefficients
Fraction of Design Space Plot
Prediction Variance
0.9
0.8
0.7
0.6
0.5
0.4
0.3
0.2
0.1
0.0
0.0
0.2
0.4
0.6
0.8
1.0
Fraction of Space

Fig.3

Fig.4

(A)

APF test_Dextrin
5
4
3
2
1
0
OD 540nm
0
8
16
24
32
40
48
Time (hr)
media1(pea)
media2(wheat)
media3(potato)
media4(potato)
media5(tomato)
media6(malt)
control1(cyg)

(B)

APF test_Glucose
5
4
3
2
1
0
OD540nm
0
8
16
24
32
40
48
Time(hr)
media7(pea)
media8(wheat)
media9(potato)
media10(potato)
media11(tomato)
media12(malt)
control2(cyg)

Fig.5

APF DoE
OD 540nm
0
1
2
3
4
5
6
7
8
9
10
Time (hr)
0
8
16
24
32
40
48
Media 1
Media 2
Media 3
Media 4
Media 5
Media 6
Media 7
Media 8
Media 9
Media 10
Media 11
Media 12
Media 13
Media 14
Media 15
Media 16

Fig.6

Distributions

Pea

| Quantiles | | |
|---|---|---|
| 100.0% | maximum | 20 |
| 99.5% | | 20 |
| 97.5% | | 20 |
| 90.0% | | 20 |
| 75.0% | quartile | 20 |
| 50.0% | median | 10 |
| 25.0% | quartile | 0 |
| 10.0% | | 0 |
| 2.5% | | 0 |
| 0.5% | | 0 |
| 0.0% | minimum | 0 |

| Summary Statistics | |
|---|---|
| Mean | 10 |
| Std Dev | 8.9442719 |
| Std Err Mean | 2.236068 |
| Upper 95% Mean | 14.766066 |
| Lower 95% Mean | 5.2339339 |
| N | 16 |
| Sum | 160 |
| Variance | 80 |
| CV | 89.442719 |
| N Missing | 0 |

Wheat

| Quantiles | | |
|---|---|---|
| 100.0% | maximum | 20 |
| 99.5% | | 20 |
| 97.5% | | 20 |
| 90.0% | | 20 |
| 75.0% | quartile | 20 |
| 50.0% | median | 10 |
| 25.0% | quartile | 0 |
| 10.0% | | 0 |
| 2.5% | | 0 |
| 0.5% | | 0 |
| 0.0% | minimum | 0 |

| Summary Statistics | |
|---|---|
| Mean | 10 |
| Std Dev | 8.9442719 |
| Std Err Mean | 2.236068 |
| Upper 95% Mean | 14.766066 |
| Lower 95% Mean | 5.2339339 |
| N | 16 |
| Sum | 160 |
| Variance | 80 |
| CV | 89.442719 |
| N Missing | 0 |

Potato

| Quantiles | | |
|---|---|---|
| 100.0% | maximum | 20 |
| 99.5% | | 20 |
| 97.5% | | 20 |
| 90.0% | | 20 |
| 75.0% | quartile | 20 |
| 50.0% | median | 5 |
| 25.0% | quartile | 0 |
| 10.0% | | 0 |
| 2.5% | | 0 |
| 0.5% | | 0 |
| 0.0% | minimum | 0 |

| Summary Statistics | |
|---|---|
| Mean | 8.125 |
| Std Dev | 9.1058589 |
| Std Err Mean | 2.2764647 |
| Upper 95% Mean | 12.97717 |
| Lower 95% Mean | 3.2728303 |
| N | 16 |
| Sum | 130 |
| Variance | 82.916667 |
| CV | 112.07211 |
| N Missing | 0 |

OD at 24hr

| Quantiles | | |
|---|---|---|
| 100.0% | maximum | 6.7621 |
| 99.5% | | 6.7621 |
| 97.5% | | 6.7621 |
| 90.0% | | 6.28407 |
| 75.0% | quartile | 5.43805 |
| 50.0% | median | 3.9533 |
| 25.0% | quartile | 2.9625375 |
| 10.0% | | 1.898503 |
| 2.5% | | 0.18351 |
| 0.5% | | 0.18351 |
| 0.0% | minimum | 0.18351 |

| Summary Statistics | |
|---|---|
| Mean | 4.0470319 |
| Std Dev | 1.6227324 |
| Std Err Mean | 0.4056831 |
| Upper 95% Mean | 4.9117249 |
| Lower 95% Mean | 3.1823388 |
| N | 16 |
| Sum | 64.75251 |
| Variance | 2.6332603 |
| CV | 40.096851 |
| N Missing | 0 |

Toxin at 48hr

| Quantiles | | |
|---|---|---|
| 100.0% | maximum | 64887.3 |
| 99.5% | | 64887.3 |
| 97.5% | | 64887.3 |
| 90.0% | | 59165.57 |
| 75.0% | quartile | 49830.55 |
| 50.0% | median | 38916.2 |
| 25.0% | quartile | 27575.425 |
| 10.0% | | 18928.92 |
| 2.5% | | 16783 |
| 0.5% | | 16783 |
| 0.0% | minimum | 16783 |

| Summary Statistics | |
|---|---|
| Mean | 39377.05 |
| Std Dev | 14221.848 |
| Std Err Mean | 3555.462 |
| Upper 95% Mean | 46955.338 |
| Lower 95% Mean | 31798.762 |
| N | 16 |
| Sum | 630032.8 |
| Variance | 202260959 |
| CV | 36.117099 |
| N Missing | 0 |

Fig.7

## Multivariate

### Correlations

| | OD at 24hr | Toxin at 48hr | Pea | Wheat | Potato |
|---|---|---|---|---|---|
| OD at 24hr | 1.0000 | 0.5888 | 0.6432 | 0.5052 | 0.0972 |
| Toxin at 48hr | 0.5888 | 1.0000 | 0.7240 | -0.1802 | -0.2051 |
| Pea | 0.6432 | 0.7240 | 1.0000 | -0.0833 | -0.0000 |
| Wheat | 0.5052 | -0.1802 | -0.0833 | 1.0000 | 0.0000 |
| Potato | 0.0972 | -0.2051 | -0.0000 | 0.0000 | 1.0000 |

The correlations are estimated by Row-wise method.

### Scatterplot Matrix

OD at 24hr
Toxin at 48hr
Pea
Wheat
Potato
6
4
2
0
70000
50000
30000
10000
20
15
10
5
0
0 2 4 6
20000
76737.5
0 5 10

Fig.8

Outlier Analysis
Mahalanobis Distances
UCL=2.963
Distance
3.0
2.5
2.0
1.5
1.0
0.5
0.0
0
5
10
15
Row Number
α = 0.05
Jackknife Distances
UCL=4.98
Distance
5
4
3
2
1
0
0
5
10
15
Row Number
α = 0.05

Fig.9

Actual by Predicted Plot
OD at 24hr Actual
OD at 24hr Predicted RMSE=0.6448 RSq=0.93
PValue=0.0024
Residual by Predicted Plot
OD at 24hr Residual
OD at 24hr Predicted
Studentized Residuals
Studentized Residual
Row Number

Effect Summary

| Source | LogWorth | PValue |
|---|---|---|
| Pea | 3.609 | 0.00025 |
| Wheat | 3.032 | 0.00093 |
| Wheat*Potato | 1.285 | 0.05184 |
| Potato*Potato | 1.254 | 0.05574 |
| Pea*Potato | 1.206 | 0.06226 |
| Pea*Wheat | 0.698 | 0.20047 |
| Pea*Pea | 0.630 | 0.23444 |
| Potato | 0.254 | 0.55690 ^ |

Externally studentized residuals with 95% simultaneous limits (Bonferroni) in red, individual limits in green.

Summary of Fit

| | |
|---|---|
| RSquare | 0.926307 |
| RSquare Adj | 0.842087 |
| Root Mean Square Error | 0.644846 |
| Mean of Response | 4.047032 |
| Observations (or Sum Wgts) | 16 |

Analysis of Variance

| Source | DF | Sum of Squares | Mean Square | F Ratio |
|---|---|---|---|---|
| Model | 8 | 36.588122 | 4.57352 | 10.9986 |
| Error | 7 | 2.910783 | 0.41583 | Prob > F |
| C. Total | 15 | 39.498905 | | 0.0024* |

Parameter Estimates

| Term | Estimate | Std Error | t Ratio | Prob>\|t\| | VIF |
|---|---|---|---|---|---|
| Intercept | 1.4354946 | 0.468677 | 3.06 | 0.0182* | . |
| Pea | 0.1281858 | 0.018766 | 6.83 | 0.0002* | 1.0163206 |
| Wheat | 0.104155 | 0.019016 | 5.48 | 0.0009* | 1.0435697 |
| Potato | -0.014199 | 0.023022 | -0.62 | 0.5569 | 1.5852246 |
| (Pea-10)*(Pea-10) | -0.00495 | 0.003805 | -1.30 | 0.2344 | 1.0444834 |
| (Pea-10)*(Wheat-10) | -0.003129 | 0.002214 | -1.41 | 0.2005 | 1.0532625 |
| (Pea-10)*(Potato-8.125) | -0.004597 | 0.002075 | -2.22 | 0.0623 | 1.001088 |
| (Wheat-10)*(Potato-8.125) | -0.004857 | 0.002076 | -2.34 | 0.0518 | 1.0020363 |
| (Potato-8.125)*(Potato-8.125) | 0.0097153 | 0.004241 | 2.29 | 0.0557 | 1.6282638 |

Fig.10

**Prediction Expression**

1.2481348977

$+ 0.130163086 \cdot Pea$

$+ 0.1043629765 \cdot Wheat$

$+ -0.01372233 \cdot Potato$

$+ (Pea - 10) \cdot ((Pea - 10) \cdot -0.004921943)$

$+ (Pea - 10) \cdot ((Wheat - 10) \cdot -0.003055008)$

$+ (Wheat - 10) \cdot ((Wheat - 10) \cdot 0.0024348894)$

$+ (Pea - 10) \cdot ((Potato - 8.125) \cdot -0.004555925)$

$+ (Wheat - 10) \cdot ((Potato - 8.125) \cdot -0.004852655)$

$+ (Potato - 8.125) \cdot ((Potato - 8.125) \cdot 0.0094239583)$

Fig.11

Interaction Profiles
OD at 24hr
Pea
Wheat
Potato
0
2
4
6
5
10
15
20

Fig.12

Actual by Predicted Plot
Conc. 48hr Actual
70000
60000
50000
40000
30000
20000
20000 30000 40000 50000 60000 70000
Conc. 48hr Predicted RMSE=9609.3 RSq=0.76
PValue=0.0482
Residual by Predicted Plot
Conc. 48hr Residual
15000
10000
5000
0
-5000
-10000
-15000
20000 30000 40000 50000 60000 70000
Conc. 48hr Predicted
Studentized Residuals
Studentized Residual
4
2
0
-2
-4
0 5 10 15
Row Number

Effect Summary

| Source | LogWorth | PValue |
|---|---|---|
| Pea | 2.366 | 0.00430 |
| Pea*Pea | 0.836 | 0.14596 |
| Pea*Potato | 0.627 | 0.23621 |
| Potato | 0.506 | 0.31221 ^ |
| Wheat*Potato | 0.466 | 0.34231 |
| Wheat*Wheat | 0.290 | 0.51260 |
| Wheat | 0.163 | 0.68740 ^ |

Externally studentized residuals with 95% simultaneous limits (Bonferroni) in red, individual limits in green.

Summary of Fit

| | |
|---|---|
| RSquare | 0.756516 |
| RSquare Adj | 0.543467 |
| Root Mean Square Error | 9609.305 |
| Mean of Response | 39377.05 |
| Observations (or Sum Wgts) | 16 |

Analysis of Variance

| Source | DF | Sum of Squares | Mean Square | F Ratio |
|---|---|---|---|---|
| Model | 7 | 2295204460 | 327886351 | 3.5509 |
| Error | 8 | 738709926 | 92338741 | Prob > F |
| C. Total | 15 | 3033914386 | | 0.0482* |

Parameter Estimates

| Term | Estimate | Std Error | t Ratio | Prob>\|t\| | VIF |
|---|---|---|---|---|---|
| Intercept | 41524.131 | 7882.841 | 5.27 | 0.0008* | . |
| Pea | 1112.5467 | 282.4505 | 3.94 | 0.0043* | 1.0367686 |
| Wheat | -117.8817 | 282.4505 | -0.42 | 0.6874 | 1.0367686 |
| Potato | -294.3954 | 272.9442 | -1.08 | 0.3122 | 1.003453 |
| (Pea-10)*(Pea-10) | -90.74848 | 56.34894 | -1.61 | 0.1460 | 1.0315941 |
| (Wheat-10)*(Wheat-10) | -38.60844 | 56.34894 | -0.69 | 0.5126 | 1.0315941 |
| (Pea-10)*(Potato-8.125) | -39.60464 | 30.92624 | -1.28 | 0.2362 | 1.0017996 |
| (Wheat-10)*(Potato-8.125) | -31.21874 | 30.92624 | -1.01 | 0.3423 | 1.0017996 |

Fig.13

**Prediction Expression**

$$41524.131261$$
$$+ 1112.5466988 \cdot Pea$$
$$+ -117.8816988 \cdot Wheat$$
$$+ -294.3953938 \cdot Potato$$
$$+ \left(Pea - 10\right) \cdot \left(\left(Pea - 10\right) \cdot -90.74847739\right)$$
$$+ \left(Wheat - 10\right) \cdot \left(\left(Wheat - 10\right) \cdot -38.60843843\right)$$
$$+ \left(Pea - 10\right) \cdot \left(\left(Potato - 8.125\right) \cdot -39.60464002\right)$$
$$+ \left(Wheat - 10\right) \cdot \left(\left(Potato - 8.125\right) \cdot -31.2187366\right)$$

Fig.14

Interaction Profiles
Conc. 48hr
70000
60000
50000
40000
30000
20000
10000
Pea
Wheat
Potato
20
0
0
5
10
15
20

Fig.15

Prediction Profiler
Pred Formula OD at 24hr
5.051972
∂Pred Formula OD at 24hr/∂Pea
0.095718
∂Pred Formula OD at 24hr/∂Wheat
0.11859
∂Pred Formula OD at 24hr/∂Potato
-0.19428
Pred Formula Toxin at 48hr
56382.77
∂Pred Formula Toxin at 48hr/∂Pea
-17.6412
∂Pred Formula Toxin at 48hr/∂Wheat
367.4212
∂Pred Formula Toxin at 48hr/∂Potato
-517.576
Desirability
0.887283
Pea
18
Wheat
7
Potato
0
Desirability
Random
Normal
Mean 18
SD 0.5
Mean 7
SD 0.5
Mean 0
SD 0.5
Simulate
Defect Rate PPM Mean SD
Pred Formula OD at 24hr 0.0012 1200 5.04746 0.65897
∂Pred Formula OD at 24hr/∂Pea 0 0 0.09573 0.00568
∂Pred Formula OD at 24hr/∂Wheat 0 0 0.11859 0.00294
∂Pred Formula OD at 24hr/∂Potato 0 0 -0.1942 0.01062
Pred Formula Toxin at 48hr 0.0004 400 56303.8 9634.75
∂Pred Formula Toxin at 48hr/∂Pea 0 0 -17.963 93.1322
∂Pred Formula Toxin at 48hr/∂Wheat 0 0 368.198 41.8595
∂Pred Formula Toxin at 48hr/∂Potato 0 0 -517.34 25.3012
All 0.0016 1600

Fig.16

(A)

Distributions
Y In Spec
5000
4000
3000
2000
1000
0
Count
0
1
Frequencies
Level Count Prob
0 1 0.00020
1 4999 0.99980
Total 5000 1.00000
N Missing 0
2 Levels

(B)

Scatterplot Matrix
Pred Formula OD at 24hr
7
6.5
6
5.5
5
4.5
4
3.5
LSL
Pred Formula Toxin at 48hr
85000
80000
75000
70000
65000
60000
55000
50000
45000
40000
35000
30000
25000
LSL
16.4 17 17.6 18.2 18.8 19.4
Pea
5.2 5.8 6.4 7 7.6 8.2
Wheat

Fig.17

Fitted Normal Distribution
Parameter Estimate Std Error Lower 95% Upper 95%
Location μ 18.010992 0.00706 17.997155 18.02483
Dispersion σ 0.4992186 0.0070666 0.4855587 0.4994104
Measures
-2*LogLikelihood 7241.2734
AICc 7245.2758
BIC 7258.3078
Goodness-of-Fit Test
A2 Prob > A2
Anderson-Darling 0.2812296 0.6730
Process Capability
Pea Capability
Histogram
LSL
USL
Density
Overall
Within
16.2 16.6 17.0 17.4 17.8 18.2 18.6 19.0 19.4 19.8
Pea
Process Summary
LSL 16.51334
USL 19.50865
N 5000
Sample Mean 18.01099
Within Sigma 0.503746
Overall Sigma 0.499219
Stability Index 0.991013
Within sigma estimated by average moving range.
Within Sigma Capability
Index Estimate Lower 95% Upper 95%
Cpk 0.991 0.965 1.017
Cpl 0.991 0.965 1.017
Cpu 0.991 0.965 1.017
Cp 0.991 0.966 1.016
Overall Sigma Capability
Index Estimate Lower 95% Upper 95%
Ppk 1.000 0.978 1.022
Ppl 1.000 0.978 1.022
Ppu 1.000 0.978 1.022
Pp 1.000 0.980 1.020
Nonconformance
Portion Observed % Expected Within % Expected Overall %
Below LSL 0.1400 0.1474 0.1350
Above USL 0.1800 0.1474 0.1350
Total Outside 0.3200 0.2949 0.2700
Process Capability
Pea Capability
Histogram
LSL_1
USL_1
Density
Overall
Within
15.5 16.0 16.5 17.0 17.5 18.0 18.5 19.0 19.5 20.0 20.5
Pea
Process Summary
LSL 15.76451
USL 20.25748
N 5000
Sample Mean 18.01099
Within Sigma 0.503746
Overall Sigma 0.499219
Stability Index 0.991013
Within sigma estimated by average moving range.
Within Sigma Capability
Index Estimate Lower 95% Upper 95%
Cpk 1.487 1.448 1.525
Cpl 1.487 1.448 1.525
Cpu 1.487 1.448 1.525
Cp 1.487 1.450 1.524
Overall Sigma Capability
Index Estimate Lower 95% Upper 95%
Ppk 1.500 1.469 1.531
Ppl 1.500 1.469 1.531
Ppu 1.500 1.469 1.531
Pp 1.500 1.471 1.529
Nonconformance
Portion Observed % Expected Within % Expected Overall %
Below LSL 0.0000 0.0004 0.0003
Above USL 0.0000 0.0004 0.0003
Total Outside 0.0000 0.0008 0.0007

Fig.18

Fitted Normal Distribution
Parameter Estimate Std Error Lower 95% Upper 95%
Location μ 6.9988494 0.0069468 6.985234 7.0124649
Dispersion σ 0.4912112 0.0070097 0.4776628 0.4913999
Measures
-2*LogLikelihood 7079.5742
AICc 7083.5766
BIC 7096.6085
Goodness-of-Fit Test
A2 Prob > A2
Anderson-Darling 0.4837168 0.2460
Process Capability
Wheat Capability
Histogram
LSL
USL
Density
Overall
Within
5.4 5.8 6.2 6.6 7.0 7.4 7.8 8.2 8.6 9.0
Wheat
Process Summary
LSL 5.525216
USL 8.472483
N 5000
Sample Mean 6.998849
Within Sigma 0.498115
Overall Sigma 0.491211
Stability Index 0.986141
Within sigma estimated by average moving range.
Within Sigma Capability
Index Estimate Lower 95% Upper 95%
Cpk 0.986 0.960 1.012
Cpl 0.986 0.960 1.012
Cpu 0.986 0.960 1.012
Cp 0.986 0.962 1.011
Overall Sigma Capability
Index Estimate Lower 95% Upper 95%
Ppk 1.000 0.978 1.022
Ppl 1.000 0.978 1.022
Ppu 1.000 0.978 1.022
Pp 1.000 0.980 1.020
Nonconformance
Portion Observed % Expected Within % Expected Overall %
Below LSL 0.1000 0.1546 0.1350
Above USL 0.2200 0.1546 0.1350
Total Outside 0.3200 0.3092 0.2700
Process Capability
Wheat Capability
Histogram
LSL_1
USL_1
Density
Overall
Within
5.0 5.5 6.0 6.5 7.0 7.5 8.0 8.5 9.0
Wheat
Process Summary
LSL 4.788399
USL 9.2093
N 5000
Sample Mean 6.998849
Within Sigma 0.498115
Overall Sigma 0.491211
Stability Index 0.986141
Within sigma estimated by average moving range.
Within Sigma Capability
Index Estimate Lower 95% Upper 95%
Cpk 1.479 1.441 1.517
Cpl 1.479 1.441 1.517
Cpu 1.479 1.441 1.517
Cp 1.479 1.442 1.516
Overall Sigma Capability
Index Estimate Lower 95% Upper 95%
Ppk 1.500 1.469 1.531
Ppl 1.500 1.469 1.531
Ppu 1.500 1.469 1.531
Pp 1.500 1.471 1.529
Nonconformance
Portion Observed % Expected Within % Expected Overall %
Below LSL 0.0000 0.0005 0.0003
Above USL 0.0200 0.0005 0.0003
Total Outside 0.0200 0.0009 0.0007

Fig.19

Profiler
Contour Profiler
Response
Contour
Current Y
Lo Limit
Hi Limit
Pred Formula OD at 24hr
4.4830071
3
∂Pred Formula OD at 24hr/∂Pea
0.1612283
∂Pred Formula OD at 24hr/∂Wheat
0.1423825
∂Pred Formula OD at 24hr/∂Potato
-0.174507
Pred Formula Toxin at 48hr
52039.703
20000
∂Pred Formula Toxin at 48hr/∂Pea
1362.664
∂Pred Formula Toxin at 48hr/∂Wheat
125.93006
∂Pred Formula Toxin at 48hr/∂Potato
-314.0132
Wheat
NOR USL
PAR LSL
PAR LSL
Pea

FIG. 20

Growth curve by APF medium
OD540nm
Time (hr)
0
4
8
12
16
20
24
28
32
36
40
44
48
0
1
2
3
4
5
6
7
8
9
Control
comparative example 1
comparative example 2
comparative example 3
comparative example 4
comparative example 5
comparative example 6
comparative example 7

FIG. 21

Toxin level for APF medium
Toxin (μg)
600
500
400
300
200
100
0
Control
Comparative Example 1
Comparative Example 2
Comparative Example 3
Comparative Example 4
Comparative Example 5
Comparative Example 6
Comparative Example 7
APF medium

## INTERNATIONAL SEARCH REPORT

International application No. **PCT/KR2024/017155**

**A. CLASSIFICATION OF SUBJECT MATTER**

**C12N 1/20**(2006.01)i; **C12N 1/38**(2006.01)i; **C12N 9/52**(2006.01)i

According to International Patent Classification (IPC) or to both national classification and IPC

**B. FIELDS SEARCHED**

Minimum documentation searched (classification system followed by classification symbols)

C12N 1/20(2006.01); C07K 14/33(2006.01); C12N 5/00(2006.01); C12N 9/64(2006.01); C12P 21/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 밀 펩톤(wheat peptone), 완두 펩톤(pea peptone), D-(+)-글루코오스(D-(+)-glucose), 효모 추출물(yeast extract), 클로스트리디움 보툴리눔(Clostridium botulinum), 배양(culture)

**C. DOCUMENTS CONSIDERED TO BE RELEVANT**

| Category* | Citation of document, with indication, where appropriate, of the relevant passages | Relevant to claim No. |
|---|---|---|
| DX | KR 10-2022-0140696 A (GALDERMA HOLDING SA et al.) 18 October 2022 (2022-10-18) See paragraph [0055]; claims 1-2, 4, 7, 12, 15 and 18; and table 1. | 1-11 |
| A | KR 10-2016-0127997 A (DAEWOONG CO., LTD.) 07 November 2016 (2016-11-07) See entire document. | 1-11 |
| A | KR 10-2016-0127999 A (DAEWOONG CO., LTD.) 07 November 2016 (2016-11-07) See entire document. | 1-11 |
| A | KR 10-2020-0114722 A (JETEMA CO., LTD.) 07 October 2020 (2020-10-07) See entire document. | 1-11 |
| A | WO 2006-096164 A1 (ALLERGAN, INC.) 14 September 2006 (2006-09-14) See entire document. | 1-11 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 February 2025** | **10 February 2025** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2024/017155**

| Patent document cited in search report | Publication date (day/month/year) | Patent family member(s) | Publication date (day/month/year) |
|---|---|---|---|
| KR 10-2022-0140696 A | 18 October 2022 | CN 114945678 A | 26 August 2022 |
| | | EP 4077706 A1 | 26 October 2022 |
| | | JP 2023-507654 A | 24 February 2023 |
| | | US 2022-0356440 A1 | 10 November 2022 |
| | | WO 2021-124296 A1 | 24 June 2021 |
| KR 10-2016-0127997 A | 07 November 2016 | CN 107109354 A | 29 August 2017 |
| | | EP 3289069 A1 | 07 March 2018 |
| | | EP 3289069 A4 | 03 October 2018 |
| | | EP 3289069 B1 | 09 December 2020 |
| | | JP 2018-500880 A | 18 January 2018 |
| | | JP 2020-022435 A | 13 February 2020 |
| | | JP 6888049 B2 | 16 June 2021 |
| | | KR 10-1723167 B1 | 05 April 2017 |
| | | US 10465179 B2 | 05 November 2019 |
| | | US 2017-0247675 A1 | 31 August 2017 |
| | | US 2018-0163193 A1 | 14 June 2018 |
| | | US 9926549 B2 | 27 March 2018 |
| | | WO 2016-175565 A1 | 03 November 2016 |
| KR 10-2016-0127999 A | 07 November 2016 | CN 107002026 A | 01 August 2017 |
| | | EP 3289071 A1 | 07 March 2018 |
| | | EP 3289071 A4 | 31 October 2018 |
| | | JP 2018-500881 A | 18 January 2018 |
| | | JP 2020-022436 A | 13 February 2020 |
| | | KR 10-1723168 B1 | 05 April 2017 |
| | | US 10308923 B2 | 04 June 2019 |
| | | US 2017-0260515 A1 | 14 September 2017 |
| | | WO 2016-175567 A1 | 03 November 2016 |
| KR 10-2020-0114722 A | 07 October 2020 | CN 113811600 A | 17 December 2021 |
| | | EP 3950930 A1 | 09 February 2022 |
| | | EP 3950930 A4 | 28 December 2022 |
| | | KR 10-2197224 B1 | 31 December 2020 |
| | | US 2022-0154133 A1 | 19 May 2022 |
| | | WO 2020-204396 A1 | 08 October 2020 |
| WO 2006-096164 A1 | 14 September 2006 | EP 1396243 A1 | 10 March 2004 |
| | | EP 1396243 B1 | 15 August 2007 |
| | | EP 1664292 A2 | 07 June 2006 |
| | | EP 1664292 B1 | 26 May 2010 |
| | | EP 1827325 A1 | 05 September 2007 |
| | | EP 1827325 B1 | 04 May 2011 |
| | | EP 1853622 A1 | 14 November 2007 |
| | | EP 1853622 B1 | 16 July 2014 |
| | | EP 1853623 A1 | 14 November 2007 |
| | | EP 1853623 B1 | 08 May 2013 |
| | | EP 1924292 A2 | 28 May 2008 |
| | | EP 2177607 A1 | 21 April 2010 |
| | | EP 2335658 A1 | 22 June 2011 |
| | | JP 2007-506427 A | 22 March 2007 |
| | | JP 2008-515582 A | 15 May 2008 |
| | | JP 2008-531046 A | 14 August 2008 |
| | | JP 2008-531047 A | 14 August 2008 |

Form PCT/ISA/210 (patent family annex) (July 2022)

**INTERNATIONAL SEARCH REPORT**
**Information on patent family members**

International application No.
**PCT/KR2024/017155**

| Patent document cited in search report | Publication date (day/month/year) | Patent family member(s) | Publication date (day/month/year) |
|---|---|---|---|
| | | JP 2009-508857 A | 05 March 2009 |
| | | JP 2012-041356 A | 01 March 2012 |
| | | JP 2012-070759 A | 12 April 2012 |
| | | JP 2012-125251 A | 05 July 2012 |
| | | JP 2013-014600 A | 24 January 2013 |
| | | JP 2015-051019 A | 19 March 2015 |
| | | JP 4712807 B2 | 29 June 2011 |
| | | JP 4913074 B2 | 11 April 2012 |
| | | JP 5006803 B2 | 22 August 2012 |
| | | JP 5122460 B2 | 16 January 2013 |
| | | JP 5518024 B2 | 11 June 2014 |
| | | JP 5567082 B2 | 06 August 2014 |
| | | KR 10-1100567 B1 | 29 December 2011 |
| | | KR 10-2006-0102330 A | 27 September 2006 |
| | | KR 10-2007-0104334 A | 25 October 2007 |
| | | KR 10-2007-0115580 A | 06 December 2007 |
| | | KR 10-2007-0116710 A | 11 December 2007 |
| | | US 2005-0069562 A1 | 31 March 2005 |
| | | US 2005-0143765 A1 | 30 June 2005 |
| | | US 2005-0143766 A1 | 30 June 2005 |
| | | US 2005-0238668 A1 | 27 October 2005 |
| | | US 2005-0238669 A1 | 27 October 2005 |
| | | US 2005-0251182 A1 | 10 November 2005 |
| | | US 2006-0228777 A1 | 12 October 2006 |
| | | US 2006-0228780 A1 | 12 October 2006 |
| | | US 2006-0240514 A1 | 26 October 2006 |
| | | US 2007-0066541 A1 | 22 March 2007 |
| | | US 2007-0111288 A1 | 17 May 2007 |
| | | US 2008-0027469 A1 | 31 January 2008 |
| | | US 2009-0022763 A1 | 22 January 2009 |
| | | US 2009-0123497 A1 | 14 May 2009 |
| | | US 2009-0124790 A1 | 14 May 2009 |
| | | US 2009-0274729 A1 | 05 November 2009 |
| | | US 2010-0222587 A1 | 02 September 2010 |
| | | US 2011-0152608 A1 | 23 June 2011 |
| | | US 2012-0149089 A1 | 14 June 2012 |
| | | US 2013-0171716 A1 | 04 July 2013 |
| | | US 2014-0039250 A1 | 06 February 2014 |
| | | US 2014-0178966 A1 | 26 June 2014 |
| | | US 2017-0342395 A1 | 30 November 2017 |
| | | US 2021-0024913 A1 | 28 January 2021 |
| | | US 2021-0079370 A1 | 18 March 2021 |
| | | US 2021-0222142 A1 | 22 July 2021 |
| | | US 2021-0238572 A1 | 05 August 2021 |
| | | US 2022-0144887 A1 | 12 May 2022 |
| | | US 7148041 B2 | 12 December 2006 |
| | | US 7160699 B2 | 09 January 2007 |
| | | US 7189541 B2 | 13 March 2007 |
| | | US 7238191 B2 | 03 July 2007 |
| | | US 7354740 B2 | 08 April 2008 |

Form PCT/ISA/210 (patent family annex) (July 2022)

**INTERNATIONAL SEARCH REPORT**
**Information on patent family members**

International application No.
**PCT/KR2024/017155**

| Patent document cited in search report | Publication date (day/month/year) | Patent family member(s) | Publication date (day/month/year) |
|---|---|---|---|
| | | US 7445914 B2 | 04 November 2008 |
| | | US 7452697 B2 | 18 November 2008 |
| | | US 7560251 B2 | 14 July 2009 |
| | | US 7714024 B2 | 11 May 2010 |
| | | US 7901419 B2 | 08 March 2011 |
| | | US 7972346 B2 | 05 July 2011 |
| | | US 8008044 B2 | 30 August 2011 |
| | | US 8012162 B2 | 06 September 2011 |
| | | US 8012716 B2 | 06 September 2011 |
| | | US 8409828 B2 | 02 April 2013 |
| | | US 8637571 B2 | 28 January 2014 |
| | | US 8841110 B2 | 23 September 2014 |
| | | US 9725705 B2 | 08 August 2017 |
| | | WO 2005-035749 A2 | 21 April 2005 |
| | | WO 2005-035749 A3 | 02 June 2005 |
| | | WO 2006-040647 A1 | 20 April 2006 |
| | | WO 2006-096163 A1 | 14 September 2006 |
| | | WO 2007-037849 A2 | 05 April 2007 |
| | | WO 2007-037849 A3 | 24 January 2008 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- KR 101339349
- KR 171729251
- KR 1020220140696

### Non-patent literature cited in the description

- **SCHANT, E. J. et al.** *Microbiol. Rev*, 1992, vol. 56, 80
- **SUGIYAMA, H.** *Microbiol. Rev*, 1980, vol. 44, 419
- **PARK. M. K. et al.** *FEMS Microbiol. Lett.*, 1990, vol. 72, 243
- **MANTECUCCO, C. et al.** *TIBS*, 1993, vol. 18, 324
- **POULAIN, B. et al.** *Proc. Natl. Acad. Sci. USA.*, 1988, vol. 85, 4090
- **SIMPSON, L. L. et al.** *Ann. Rev. Pharmaeol. Toxicol*, 1986, vol. 26, 427
- **BINZ, T. et al.** *J. Biol. Chem.*, 1994, vol. 265, 9153
- **PRUSINER SB**. *Alzheimer Dis Assoc Disord.*, 1989, vol. 3, 52-78